(19) Europäisches Patentamt
European Patent Office
Office européen des brevets

(11) Veröffentlichungsnummer : **0 012 158**
**B1**

(12) # EUROPÄISCHE PATENTSCHRIFT

(45) Veröffentlichungstag der Patentschrift : **15.08.84**

(21) Anmeldenummer : **79103212.1**

(22) Anmeldetag : **30.08.79**

(51) Int. Cl.³ : **A 01 N 25/32**, C 07 C131/00, C 07 C143/78, C 07 C121/84, C 07 D521/00

(54) **Oximderivate und ihre Anwendung zum Schutz von Pflanzenkulturen.**

(30) Priorität : **01.09.78 CH 9255/78**

(43) Veröffentlichungstag der Anmeldung : **25.06.80 Patentblatt 80/13**

(45) Bekanntmachung des Hinweises auf die Patenterteilung : **15.08.84 Patentblatt 84/33**

(84) Benannte Vertragsstaaten :
**AT BE CH DE FR GB IT NL**

(56) Entgegenhaltungen :
**EP-A- 0 007 678**
**BE-A- 646 457**
**BE-A- 715 017**
**CH-A- 366 690**
**CH-A- 535 210**
**DE-A- 1 670 157**
**DE-A- 2 257 062**
**DE-A- 2 262 189**
**DE-A- 2 808 317**
**DE-B- 1 567 075**
**FR-A- 1 266 033**
**FR-A- 2 100 751**
**FR-A- 2 322 861**
**LU-A- 78 814**
**US-A- 3 481 944**
**US-A- 3 629 472**
**US-A- 3 681 386**
**US-A- 4 032 558**
**US-A- 4 061 749**
**CHEMICAL ABSTRACTS, Band 86, Nr. 11, 14. März 1977, Seite 595, Nr. 72439y, Columbus, Ohio, U.S.A.**

(73) Patentinhaber : **CIBA-GEIGY AG**
**Postfach**
**CH-4002 Basel (CH)**

(72) Erfinder : **Lukaszczyk, Alfons**
**Bruderholzweg 38**
**CH-4053 Basel (CH)**
Erfinder : **Martin, Henry, Dr.**
**Jupiterstrasse 17**
**CH-4123 Allschwil (CH)**
Erfinder : **Diel, Peter J., Dr.**
**Unterwartweg 13**
**CH-4132 Muttenz (CH)**
Erfinder : **Föry, Werner, Dr.**
**Benkenstrasse 65**
**CH-4054 Basel (CH)**
Erfinder : **Gätzi, Karl, Dr.**
**Realpstrasse 69**
**CH-4054 Basel (CH)**
Erfinder : **Kristinsson, Haukur, Dr.**
**Kreuzackerweg 19**
**CH-4103 Bottmingen (CH)**
Erfinder : **Müller, Beat, Dr.**
**Stockackerstrasse 3**
**CH-4153 Reinach (CH)**
Erfinder : **Muntwyler, René, Dr.**
**Hutmattweg 6**
**CH-4114 Hofstetten (CH)**
Erfinder : **Pachlatko, Johannes Paul, Dr.**
**Waldhofstrasse 40**
**CH-4310 Rheinfelden (CH)**
Erfinder : **Rempfler, Hermann, Dr.**
**Brücklismattstrasse 16**
**CH-4107 Ettingen (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 012 158 B1**

Erfinder : **Schurter, Rolf, Dr.**
**Holzmattstrasse 45**
**CH-4102 Binningen (CH)**
Erfinder : **Szczepanski, Henry, Dr.**
**Waldshuterstrasse 55**
**CH-4310 Rheinfelden (CH)**

Vertreter : **Zumstein, Fritz sen., Dr. et al**
**Bräuhausstrasse 4**
**D-8000 München 2 (DE)**

**Beschreibung**

Die vorliegende Erfindung betrifft Oxim-Derivate der allgemeinen Formel I

$$\begin{array}{l} Ar - C - X \\ \qquad \| \\ \qquad N - O - Q \end{array} \qquad (I)$$

neue Mittel auf Basis dieser Verbindungen und ferner die Verwendung solcher Verbindungen oder sie enthaltender Mittel zum Schutz von Kulturpflanzen vor der phytotoxischen Wirkung von aggressiven Agrarchemikalien.

Die Wirkstoffe entsprechen der Formel I, worin

Ar einen 5-gliedrigen, ungesättigten, gegebenenfalls benzokondensierten heterocyclischen Ring welcher 2 oder 3 Heteroatome aus der Gruppe N, O und enthält, von denen mindestens eines ein Stickstoffatom ist, bedeutet, der am heterocyclischen oder aromatischen Teil durch $R_1$, $R_2$ oder $R_3$ substituiert ist und/oder gegebenenfalls durch Oxo oder Thiono substituiert ist, wobie $R_1$ für Wasserstoft, Halogen, $C_1$-$C_4$-Alkyl oder $C_1$-$C_4$-Alkoxy, $R_2$ für Wasserstoff, Halogen, Nitro, $C_1$-$C_4$-Alkyl, Halogen-$C_1$-$C_4$-Alkyl, $C_1$-$C_4$-Alkoxy und $R_3$ für Wasserstoff oder Phenyl stehen oder, wenn als Heterocyclus ein Thiadiazol vorliegt, $R_1$ und $R_2$ für Wasserstoff und $R_3$ für $C_1$-$C_4$-Alkoxy, $C_1$-$C_4$-Alkylthio, $C_1$-$C_4$-Alkylsulfinyl, $C_1$-$C_4$-Alkylsulfonyl oder Phenyl stehen,

X Wasserstoff, CN, Halogen, $C_1$-$C_4$-Alkyl oder $C_1$-$C_4$-Alkoxycarbonyl sind und

Q Wasserstoff, ein Metallkation, $C_1$-$C_4$-Alkyl, welches gegebenenfalls durch Cyan oder Carbamoyl substituiert ist, $C_2$-$C_4$-Alkenyl, $C_2$-$C_4$-Alkinyl, $C_1$-$C_4$-Alkylcarbonyl, Halogen-$C_1$-$C_4$-Alkylcarbonyl, $C_2$-$C_4$-Alkenylcarbonyl, $C_1$-$C_4$-Alkoxycarbonyl, $C_1$-$C_4$-Alkylthio-carbonyl, $C_1$-$C_4$-Alkylsulfonyl, $C_1$-$C_4$-Alkoxy-carbonyl-$C_1$-$C_4$-Alkyl, Furanoyl, Benzoyl, welches gegebenenfalls durch Substituenten der Gruppe Methyl, Methoxy, Halogen oder Nitro ein- bis zweifach substituiert ist, Benzylcarbonyl, Aminocarbonyl, welches am Stickstoffatom gegebenenfalls ein- oder zweimal durch $C_1$-$C_4$-Alkyl, $C_2$-$C_4$-Alkenyl, Halogen-$C_1$-$C_4$-Alkyl oder $C_1$-$C_4$- oder $C_1$-$C_4$-Alkoxy oder einmal durch Phenyl, welches gegebenenfalls Trifluormethyl und/oder Halogen als Substituenten trägt, substituiert ist, oder Phenylsulfonyl bedeuten.

In der Formel I ist unter Halogen Fluor, Chlor, Brom oder Jod zu verstehen.

Der Ausdruck Alkyl allein oder als Teil eines Substituenten umfasst verzweigte oder unverzweigte $C_1$- bis $C_4$-Alkylgruppen ; Beispiele sind Methyl, Aethyl, Propyl, Isopropyl, Butyl, Isobutyl, sec. Butyl und tert. Butyl.

Als Beispiele für den Rest Ar seien aus der Reihe der monocyclischen Ringe Imidazol, Pyrazol, Oxazol, Thiazol, Thiadiazol und Triazol.

Aus der bicyclischen Reihe die vorstehend aufgezählten Ringsysteme in Kombination mit einem ankondensierten Benzolring (z. B. Benzthiazol, Benzoxazol) genannt.

Durch den Einbau von Oxo und/oder Thiono entstehen weitere heterocyclische Ringsysteme. N-Heterocyclen umfassen auch N-Oxide.

Als Metallsalze sind Kationen der I. bis IV. Gruppen des Periodensystems sowie Schwermetallsalze zu verstehen. Beispiele sind Na, K, Ca, Mg, Al, Zn, Cu, Fe, Mn, Co, Ni.

Oxime der Formel I eignen sich hervorragend, Kulturpflanzen wie Reis, Mais, Getreidearten (Kulturhirse, Weizen, Roggen, Gerste, Hafer), vor dem Angriff von pflanzenagressiven Agrarchemikalien, insbesondere von Herbiziden verschiedenster Stoffklassen, zu schützen, sofern diese nicht oder nicht genügend selektiv wirken, also neben den zu bekämpfenden Unkräutern auch die Kulturpflanzen mehr oder weniger schädigen.

Als Gegenmittel oder Antidote sind schon verschiedene Stoffe vorgeschlagen worden, welche befahigt sind, die schädigenden Wirkung eines Herbizids auf die Kulturpflanze spezifisch zu antagonisieren, d. h. die Kulturpflanze zu schützen, ohne dabei die Herbizidwirkung auf die zu bekämpfenden Unkräuter merklich zu beeinflussen. Dabei kann ein solches Gegenmittel, auch Safener genannt, je nach seinen Eigenschaften zur Vorbehandlung des Saatgutes der Kulturpflanze (Beizung des Samens oder der Stecklinge) oder vor der Saat in die Saaufurchen oder als Tankmischung für sich allein oder zusammen mit dem Herbizid vor oder nach dem Auflaufen der Pflanzen verwendet werden.

So beschreibt die GB-PS 1'277'557 die Behandlung von Samen bzw. Sprösslingen von Weizen und Sorghum mit gewissen Oxamsäureestern und Amiden vor dem Angriff durch N-Methoxymethyl-2',6'-diäthyl-chloracetanilid (Alachlor). Andere Literaturstellen (DE-OS 1'952'910, DE-OS 2'245'471, FR-PS 2'021'611) schlagen Gegenmittel zur Behandlung von Getreide, Mais- und Reis-Samen zum Schutz gegen den Angriff herbizider Thiolcarbamate vor. In der DE-AS 1'567'075 und der US-PS 3'131'509 werden Hydroxyamino-acetanilide und Hydantoine für den Schutz von Getreidesamen gegenüber Carbamaten wie IPC, CIPC etc. vorgeschlagen. In der weiteren Entwicklung haben sich alle diese Präparate jedoch als ungenügend erwiesen. Weiterhin ist Phenylglyoxylonitril-2-oxim-cyanomethyläther als Gegen mittel für Kulturpflanzen schädigende Herbizide in FR 2'322'861 beschrieben worden.

Oximderivate und ihr Einsatz als selektive Herbizide, Nematozide, Acarizide, Insektizide, Bakterizide,

Fungizide, Pharmazeutika und Ausgangsproduchte für optische Aufheller sind ferner bekannt aus Chem. Abstr. 86 (1977), 72439, US-PS 3'681'386, US-PS 4'061'749 BE-PS 715'017, CH-PS 366'690, US-PS 3'629'472, FR-PS 1'266'033, DE-OS 2'257'062, FR-PS 2'100'751, CH-PS 535'210 und US-PS 4'032'558.

Eine Gruppe bevorzugter Verbindungen sind solche der Formel

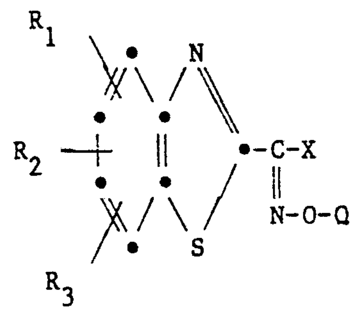

worin $R_1$, $R_2$ und $R_3$ die für Formel I angegebenen Bedeutungen haben,

X CN oder $C_1$-$C_4$-Alkoxycarbonyl und

Q $C_1$-$C_4$-Alkyl, welches gegebenenfalls durch Cyan substituiert ist, $C_2$-$C_4$-Alkenyl, $C_2$-$C_4$-Alkinyl, $C_1$-$C_4$-Alkylcarbonyl, $C_2$-$C_4$-Alkenylcarbonyl, $C_1$-$C_4$-Alkylsulphonyl, Benzoyl, welches gegebenenfalls durch Halogen substituiert ist, oder Aminocarbonyl, welches am Stickstoffatom gegebenenfalls ein- oder zweimal durch $C_1$-$C_4$-Alkyl oder $C_2$-$C_4$-Alkenyl oder einmal durch Phenyl, welches gegebenenfalls Trifluormethyl und/oder Halogen als Substituenten trägt, substituiert ist, bedeuten.

Besonders bevorzugt sind jene Verbindungen, bei denen $R_1$, $R_2$ und $R_3$ Wasserstoff bedeuten.

Eine andere Untergruppe bevorzugter Verbindungen sind solche der Formel I

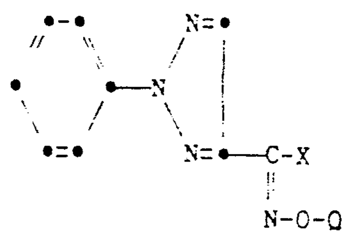

worin

X für Wasserstoff und

Q für Wasserstoff oder Aminocarbonyl, welches am Stickstoffatom durch $C_1$-$C_4$-Alkyl oder Halogen-$C_1$-$C_4$-Alkyl mono- oder disubstituiert ist, stehen.

Eine andere Untergruppe bevorzugter Verbindungen sind solche der Formel I, worin

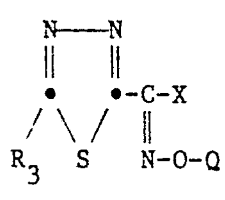

worin $R_3$

$C_1$-$C_4$-Alkoxy, $C_1$-$C_4$-Alkylthio, $C_1$-$C_4$-Alkylsulfinyl, $C_1$-$C_4$-Alkylsulfonyl oder Phenyl, X Wasserstoff, Cyan, Halogen, $C_1$-$C_4$-Alkyl oder $C_1$-$C_4$-Alkoxycarbonyl und Q Wasserstoff oder $C_1$-$C_4$ Alkyl, welches gegebenenfalls durch Cyan substituiert ist, bedeuten.

Besonders bevorzugt sind jene Verbindungen, bei denen X CN, Halogen oder $C_1$-$C_4$-Alkoxycarbonyl bedeutet.

Eine andere Untergruppe bevorzugter Verbindungen sind solche der Formel I

(Siehe Formel, Seite 4 f.)

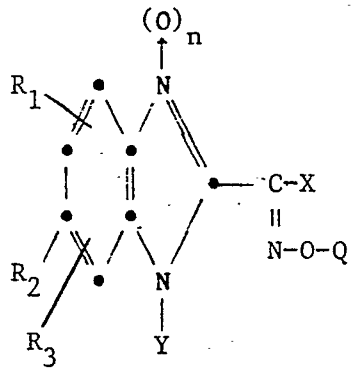

worin n die Zahl 0 oder 1, $R_1$ und $R_3$ Wasserstoff, $R_2$ Wasserstoff oder Halogen, $C_1$-$C_4$-Alkyl, $C_1$-$C_4$-Alkoxycarbonyl oder Phenyl, X Wasserstoff, Cyan, Halogen oder $C_1$-$C_4$-Alkyl und Q Wasserstoff, $C_1$-$C_4$-Alkylcarbonyl, Benzoyl, welches gegebenenfalls durch Halogen substituiert ist, oder Aminocarbonyl, welches am Stickstoff atom gegebenenfalls ein- oder zweimal durch $C_1$-$C_4$-Alkyl, oder einmal durch Phenyl, welches gegebenenfalls Halogen als Substituenten trägt, substituiert bedeuten.

Hervorzuheben sind Oximderivate aus der nachfolgenden Gruppe :

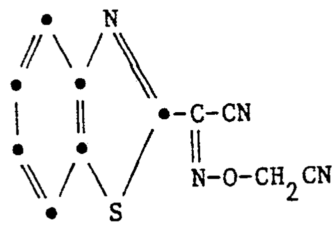

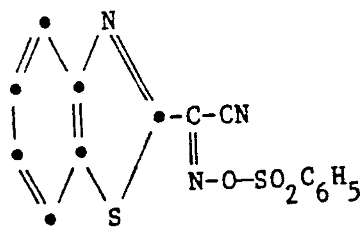

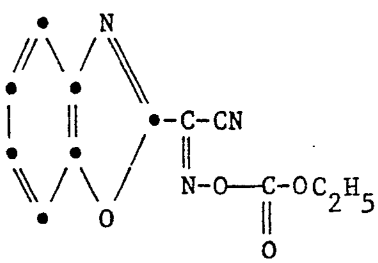

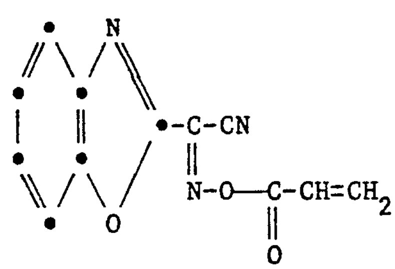

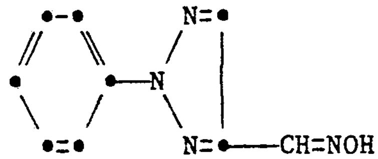

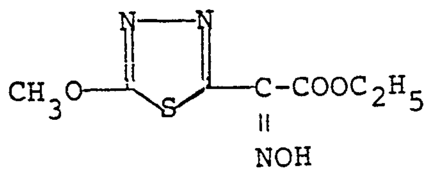

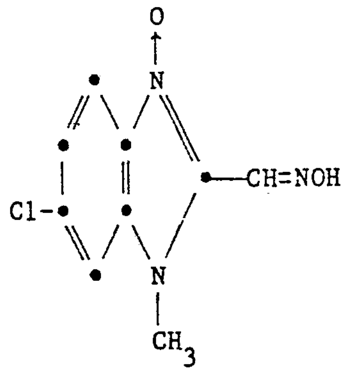

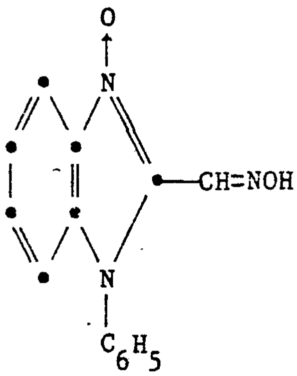

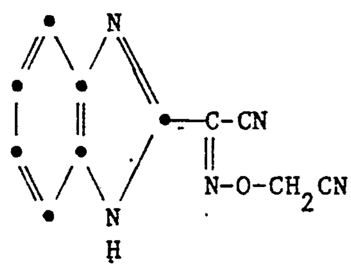

und

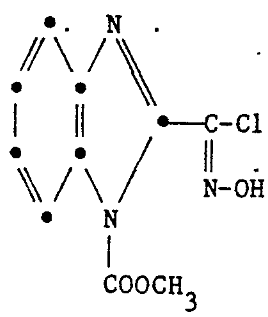

Ueberraschenderweise besitzen Oxime der Formel I die Eigenschaft, Kulturpflanzen vor dem Angriff pflanzenaggressiver Agrarchemikalien zu schützen, insbesondere vor Herbiziden der verschiedensten Stoffklassen, darunter 1,3,5-Triazine, 1,2,4-Triazinone, Phenylharnstoffderivate, Carbamate, Thiolcarbamate, Phenoxyessigsäureester, Phenoxypropionsäureestern, Halogenacetanilide, Halogenphenoxyessigsäureester, subst, Phenoxyphenoxyessigsäureester und -propionsäureester, subst. Pyridinoxyphenoxy-essigsäureester und -propionsäureester, und Benzoesäurederivate, sofern diese nicht oder ungenugend kulturentolerant sind.

Ein solches Gegenmittel oder Antidote der Formel I kann je nach Anwendungszweck zur Vorbehandlung des Saatgutes der Kulturpflanze (Beizung des Samens oder der Stecklinge) eingesetzt oder vor oder nach der Saat in den Erdboden gegeben werden oder aber für sich allein oder zusammen mit dem Herbizid vor oder nach dem Auflaufen der Pflanzen appliziert werden. Die Behandlung der Pflanze oder des Saatguts mit dem Antidote kann daher grundsätzlich unabhängig vom Zeitpunkt der Applikation der phytotoxischen Chemikalie erfolgen. Sie kann jedoch auch gleichzeitig durchgeführt werden (Tankmischung). Vorauflauf-Behandlung schliesst sowohl die Behandlung der Anbaufläche vor der Aussaat (ppi = « pre plant incorporation ») als auch die Behandlung der angesäten, aber noch nicht bewachsenen Anbauflächen ein.

Die Aufwandmengen des Antidotes im Verhältnis zum Herbizid richten sich weitgehend nach der Anwendungsart. Sofern eine Feldbehandlung vorgenommen wird, verhalten sich die Mengen von Antidote der Formel I zu phytotoxischer Chemikalie wie 1 : 100 bis 5 : 1, bevorzugt 1 : 20 bis 1 : 1. Bei Samenbeizung und ähnlichen gezielten Schutzmassnahmen werden jedoch weit geringere Mengen Antidote im Vergleich mit den z. B. später pro Hektar Anbaufläche verwendeten Herbizidmengen benötigt (z. B. ca. 1 : 3 000 bis (1 : 1 000). In der Regel stehen protektive Massnahmen wie Samenbeizung mit einem Antidote der Formel I und mögliche spätere Feldbehandlung mit Agrarchemikalien nur in losem Zusammenhang. Vorbehandeltes Saat- und Pflanzengut kann später in Landwirtschaft. Gartenbau und Forstwirtschaft mit unterschiedlichen Chemikalien in Berührung kommen.

Die Erfindung betrifft sowohl Mittel, welche diese Oximäther der Formel I zusammen mit Herbiziden enthalten, als auch Mittel, welche diese Oximäther der Formel I als einzige Aktivkomponente enthalten. Pflanzenschutzmittel, die ein Gegenmittel der Formel I (auch Antidote oder Saferer genannt) enthalten, können ohne gleichzeitige Anwesenheit des abzuschwächenden Herbizids (oder einer anderen aggressiven Agrarchemikalie) hergestellt, in den Handel gebracht oder verwendet werden. Eine wichtige Anwendungsmöglichkeit ist die Saatbeizung, die zu einem von der Anwendung der Agrarchemikalie (z. B. Herbizide) ganz unabhängigen Zeitpunkt vorgenommen wird. Ein anderes Einsatzgebiet ist die Behandlung eines Bodens, in dem noch Reste eines Herbizids der letzten Anbausaison vorhanden sind, das die vorgesehene neue Pflanzenkultur schädigen könnte.

Die Antidote-Eigenschaft ist eine von der Kulturpflanze und von der in der Wirkung selektiv abzuschwächenden Agrarchemikalie unabhängige Stoffeigenschaft, die einem Präparat der Formel I inhärent ist, jedoch erst beim Zusammenwirken der 3 Komponenten Antidote-Agrarchemikalie-Pflanze sichtbar zu Tage tritt. Aehnlich einer pestizid wirkenden Chemikalie, deren Pestizid-Wirkung erst bei Anwesenheit eines Schädlings sichtbar wird, erfordert auch der Nachweis der Safener-Wirkung die Anwesenheit der an der Wirkung beteiligten anderen beiden Komponenten, nämlich der Agrarchemikalie (z. B. dem Herbizid) und der Kulturpflanze. Dies unterscheidet ein formuliertes Safener-Mittel von einem synergistisch wirkenden Zwei- oder Dreikomponentengemisch, bei dem gleichzeitig alle Wirkstoffkomponenten vorliegen und sämtlich in die gleiche Wirkungsrichtung zielen.

Kulturpflanzen seien im Rahmen vorliegender Erfindung alle Pflanzen, die in irgendeiner Form Ertragsstoffe produzieren (Samen, Wurzeln, Stengel, Knollen, Blätter, Blüten, Inhaltsstoffe wie Oele, Zucker, Stärke, Eiweiss etc.) und zu diesem Zweck angebaut und gehegt werden. Zu diesen Pflanzen gehören beispielsweise sämtliche Getreidearten, Mais, Reis, Edelhirse, Soja, Bohnen, Erbsen, Kartoffeln, Gemüse, Baumwolle, Zuckerrüben, Zuckerrohr, Erdnüsse, Tabak, Hopfen, dann jedoch auch Zierpflanzen, Obstbäume sowie Bananen-, Kakao- und Naturkautschuk-Gewächse. Diese Aufzählung stellt keine Limitierung dar. Grundsätzlich lässt sich ein Antidote überall dort einsetzen, wo eine Kulturpflanze vor der Phytotoxizität einer Chemikalie geschützt werden soll.

Die Erfindung bezieht sich auch auf ein Verfahren zum Schutz von Kulturpflanzen vor aggressiven (phytotoxischen) Agrarchemikalien, indem ein als Antidote wirkendes Oximderivat der Formel I wahlweise vor oder nach Applikation der Agrarchemikalie oder auch gleichzeitig mit der Agrarchemikalie angewendet wird.

Die Erfindung bezieht sich ferner auf das Vermehrungsgut solcher Kulturpflanzen, das protektiv mit einem Oximderivat der Formel I behandelt wurde. Unter dem Begriff « Vermehrungsgut » sind alle generativen Pflanzenteile zu verstehen, die zur Vermehrung der Kulturpflanze eingesetzt werden können. Dazu zählen Samenkörner (Saatgut im engeren Sinne), Wurzeln, Früchte, Knollen, Rhizome, Stengelteile, Zweige (Stecklings) und andere Pflanzenteile. Es zählen aber auch angekeimte Pflanzen und Jungpflanzen dazu, die nach der Ankeimung oder dem Auflaufen weiterverpflanzt werden sollen. Solche Jungpflanzen lassen sich durch eine vollständige oder partielle Tauchbehandlung vor dem Weiterverpflanzen gezielt schützen.

Die unter den Umfang der Formel I fallenden freien Oxime lassen sich nach an sich bekannten Methoden der Oximierung von Verbindungen der Formel II

$$Ar—CH_2—X \qquad (II)$$

(mit der für Formel I gegebenen Bedeutung) durch Reaktion mit salpetriger Säure ($HNO_2$) bzw. einem organischen oder anorganischen Nitrit darstellen. Sie lassen sich (in Abhängigkeit von Substituenten X) auch aus Keto-Verbindungen der Formel III

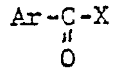 (III)

durch Reaktion mit Hydroxylamin gewinnen.

Die so gewonnenen freien Oxime lassen sich dann, sofern gewünscht, in die übrigen von der Formel I umfassten Derivate überführen, und zwar wahlweise

a) mit Basen in die Salze überführen, oder

b) mit Säuren oder Säurehalogeniden acylieren, oder

c) mit Isocyanaten oder Carbamoylhalogeniden in Oximcarbamate verwandeln, oder

d) mit Halogenkohlensäure(thio) estern in (Thiolo) Carbonate überführen, oder

e) mit halogenabspaltenden Resten in sonstige, unter Q aufgeführte Oximäther umwandeln, oder f) mit $COCl_2$ in Chloride überführen, die mit Aminen Carbamate bilden. Sofern der Reaktionspartner einen halogenabspaltenden Rest trägt, wird das freie Oxim vorzugsweise in Form eines Salzes, vorzugsweise eines Alkalisalzes, eingesetzt.

Es sei zur Herstellungsmethodik als Literatur zitiert : DT-OS 2,312,956 ; DT-OS 2,350,910 ; « Berichte der deutschen chem. Gesellschaft » *42,* S. 738 ff. [1909] ; J.F. prakt. Chemie *66,* S. 353 ; Liebigs Ann. *250,* 165 ; Organic Reactions 1953, Bd. 7, Seite 343 und 373.

Als Lösungsmittel zur Gewinnung der Verbindungen der Formel I eignen sich prinzipiell alle Vertreter, die sich unter den Bedingungen der Reaktion indifferent erhalten. Beispielsweise Kohlenwasserstoffe, vor allem aber polare Lösungsmittel wie Acetonitril, Dioxan, Cellosolve, Dimethylformamid, wasserfreie Essigsäure, Pyridin usw.

Die Temperaturen liegen im Bereich von – 20° bis ca. 150° bevorzugt zwischen 20° und 60°.

Als halogenwasserstoffabspaltende Mittel können Basen wie tert. Amine (Triäthylamin, Triäthylendiamin, N-Methylpiperidin, N-Methylmorpholin, Dimethylanilin) eingesetzt werden. In einigen Fällen genügt hierfür eine Suspendierung von wasserfreier $Na_2CO_3$ oder wasserfreier $K_2CO_3$ in wasserfreiem Reaktionsmedium oder von NaOH-Lösungen unter Phasentransfer-Bedingungen.

Oxime liegen in zwei stereoisomeren Formen, der syn. und anti-Form, vor. Alle genannten Endprodukte entsprechen der Formel I und können in beiden Formen rein oder als Gemische vorliegen. Im Rahmen der vorliegenden Beschreibung sind demgemäss beide stereoisomere Formen für sich und als Gemische in beliebigem gegenseitigen Mischungsverhältnis zu verstehen.

Die folgenden Beispiele illustrieren die Herstellung von Oximderivaten der Formel I. Die Temperaturen sind dort und in der nachfolgenden Tabelle in Celsiusgraden angegeben.

### Beispiel 1

Herstellung von

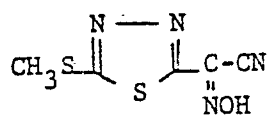

[Verb. Nr. 204]

α-[5-Methylthio-1,3,4-thiadiazol-2-yl]-α-oximinoacetonitril

17,1 g (0,1 Mole) 2-Cyanmethyl-5-methylthio-1,3,4-thiadiazol werden in 75 ml Eisessig vorgelegt. Man tropft eine Lösung von 7,8 g (0,11 Mole) $NaNO_2$ in 20 ml Wasser dazu und lässt 1 Std. rühren. Dann versetzt man die Lösung mit 200 ml Wasser und filtriert. Der Rückstand wird mit Wasser gewaschen : 19 g (95 % d. Th.) Endprodukt, Smp. > 200 °C (Zers.)

### Beispiel 2

Herstellung von

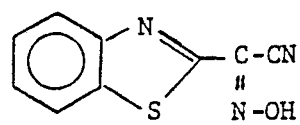

[Verb. 66]

α-[Benzthiazol-2-yl]-α-oximino-acetonitril

Zu 17,4 g (0,1 Molen) 2-Cyanomethyl-benzthiazol in 50 ml Eisessig tropft man bei Raumtemperatur eine Lösung von 7,8 g (0,1 Molen) $NaNO_2$ in 20 ml Wasser. Man filtriert nach 2 Std. das Endprodukt und wäscht es mit Wasser : 19,5 g (96 % d. Th), Smp. > 170° (Zers.)

### Beispiel 3

Herstellung von

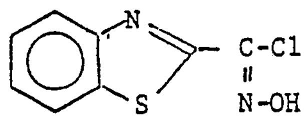

[Verb. 82]

Benzthiazol-2-yl-hydroxamsäurechlorid

36,7 g (0,2 Mole) 2-Chlormethylbenzthiazol und 23,4 g (0,2 Mole) Isopentylnitrit werden in 200 ml Dioxan vorgelegt. Unter Kühlung (< 50 °C) werden während 30 Min. ca. 0,4 Mole Chlorwasserstoffgas eingeleitet. Nach 2 Std. Rühren wird abgekühlt und das Endprodukt filtriert, mit wenig Dioxan und dann mit Petroläther gewaschen : 39,5 g (93 % d. Th), Smp. 192°.

Beispiel 4

Herstellung von

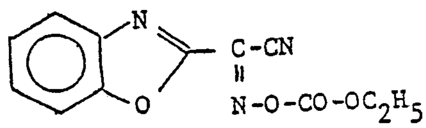

[Verb. 111]

α-[Benzoxazol-2-yl]-α-[O-äthoxycarbonyl-oximino]-acetonitril

Man tropft 12 g (0,11 Mole) Chlorameisensäureäthylester unter Eiskühlung zu einer Lösung von 18,7 g (0,1 Mole) α-[Benzoxazol-2-yl]-α-oximino-acetonitril in 120 ml Pyridin. Nach 3 Std. Rühren wird das Gemisch auf 1 Liter Eiswasser gegossen und der Rückstand abfiltriert und mit Wasser gewaschen : 23 g (89 % d. Th.) Endprodukt, das aus Acetonitril umkristallisiert wird : Smp. 187 °C.

Auf diese Art oder nach einer der vorstehend angegebenen Methoden lassen sich folgende Verbindungen der Formel

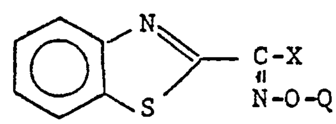

herstellen :

| Verb. Nr. | X | Q | Schmelzpunkt |
|---|---|---|---|
| 5 | -CN | H | 170° (Z) |
| 6 | CN | $Na^+$ | (fest) |
| 7 | CN | $-CON(CH_3)_2$ | 200° |
| 8 | CN | $-COCH_3$ | 168° |
| 9 | CN | $-CO-OC_3H_7$(iso) | 186° |
| 10 | CN | $-SO_2-CH_3$ | 202° |
| 11 | CN | $-CO-NHCH_3$ | 164° |
| 12 | CN | $-CO-CH=CH-CH_3$ | 75° |
| 13 | CN | $CH_3$ | 152° |
| 14 | CN | $-CH_2-C\equiv CH$ | 118° |
| 15 | $-COOC_2H_5$ | H | 120° (Z) |
| 16 | -CN | $-CH_2-CN$ | 207° |
| 17 | CN | $-CONH-C_6H_4-Cl(4)$ | 194° |
| 18 | CN | $-CO-C_6H_3Cl_2(2,4)$ | 190° (Z) |
| 19 | CN | $-CONH-C_6H_3Cl(3)-CF_3(4)$ | 206° |
| 20 | CN | $-CO-C_6H_4Cl(4)$ | 190° |
| 21 | Cl | H | 192° |

(Fortsetzung)

| Verb. Nr. | X | Q | Schmelzpunkt |
|---|---|---|---|
| 22 | Cl | $-CONHCH_3$ | |
| 23 | Cl | $-CONH-C_6H_5$ | |
| 24 | $COOCH_3$ | H | 124° (Z) |
| 25 | CN | $-CONH-CH_2-CH=CH_2$ | 130° |
| 26 | CN | $-SO_2-C_6H_5$ | 204° |

sowie folgende Verbindungen der Formel

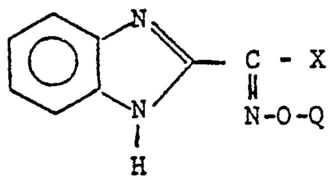

| Verb. Nr. | X | Q | Schmelzpunkt |
|---|---|---|---|
| 27 | CN | H | > 270° (Z) |
| 28 | CN | $Na^+$ | (fest) |
| 29 | CN | $-CON(CH_3)_2$ | > 240° (Z) |
| 30 | CN | $-SO_2CH_3$ | > 210° (Z) |
| 31 | CN | $-CONHCH_3$ | > 210° (Z) |
| 32 | CN | $-COCH_3$ | 210° |
| 33 | CN | $-CH_2-CN$ | 179° |
| 34 | CN | $-CO-NHC_2H_5$ | 285° |
| 35 | CN | $-CO-NHC_3H_7$(n) | 233° |
| 36 | CN | $-CO-NHC_3H_7$(iso) | 112° (Z) |
| 37 | CN | $-CO-NHC_4H_9$(n) | 238° (Z) |
| 38 | CN | $-CO-NHC_4H_9$(tert.) | 310° (Z) |
| 39 | CN | $-CO-C_6H_3Cl_2$(3,4) | > 250° (Z) |
| 40 | CN | $-CO-NHC_6H_4Cl$(4) | > 200° (Z) |
| 41 | Cl | $-CH_2CN$ | |
| 42 | Cl | $-CH_2-C\equiv CH$ | |
| 43 | Cl | $-CH_2-CH=CH_2$ | |
| 44 | Cl | $-CH_2-CH=CH-CH_3$ | |
| 45 | CN | $-CH_2-CH=CH-CH_3$ | |
| 46 | $CH_3$ | $-CH_2-CH=CH-CH_3$ | |
| 47 | CN | $-CH_2-CONH_2$ | |

sowie folgende Verbindungen der Formel

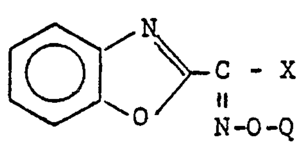

(Fortsetzung)

| Verb. Nr. | X | Q | Schmelz-punkt |
|---|---|---|---|
| 48 | CN | H | 220° (Z) |
| 49 | CN | $-CO-CH_3$ | 180° |
| 50 | CN | $-CO-CH_2Cl$ | |
| 51 | CN | $-CO-OC_2H_5$ | 199° |
| 52 | CN | $-CON(CH_3)_2$ | 212° |
| 53 | CN | $-CO-NHCH_3$ | >170° (Z) |
| 54 | CN | $-SO_2CH_3$ | 207° |
| 55 | CN | $-CH_2-C \equiv CH$ | 107° |
| 56 | $-COOC_2H_5$ | H | 134° (Z) |
| 57 | $-COOCH_3$ | H | 137° (Z) |
| 58 | CN | $CH_3$ | 125° |
| 59 | CN | $-CO-OC_3H_7$(iso) | 179° |
| 60 | CN | $-CO-C_4H_9$(n) | (fest) |
| 61 | CN | $-CO-N(CH_3)-OCH_3$ | 171° |
| 62 | CN | $-CO-OCH_3$ | 173° |
| 63 | CN | $-COC_3H_7$( n ) | 96° |
| 64 | CN | $-CO-CH=CH-CH_3$ | 153° |
| 65 | CN | $-CO-CH=CH_2$ | |
| 66 | CN | $-CO-SC_2H_5$ | 154° |
| 67 | CN | $-CO-NH-CH_2CH_2Cl$ | 150-155° |
| 68 | CN | $-CO-NH-CH_2Cl$ | 165-168° |
| 69 | CN | $-CH_2-CN$ | 164° |
| 70 | CN | 2-Furanoyl | 230° |
| 71 | CN | $-CO-C_6H_3Cl_2$(3,4) | >210° (Z) |
| 72 | CN | $-CO-NH-C_6H_3Cl_2$(3,5) | 197° |
| 73 | CN | $-CO-C_6H_4Cl$(2) | 145° |
| 74 | CN | 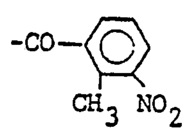 | 200° |
| 75 | CN | $-CO-C_6H_3Cl_2$(3,5) | 183° |
| 76 | CN | $-CO-C_6H_4-CH_3$(2) | 182° |
| 77 | CN | $-CO-C_6H_4-OCH_3$(2) | 166° |
| 78 | Cl | H | >180° (Z) |
| 79 | Cl | $-CONH-C_6H_5$ | 167° |
| 80 | Cl | $-CONH-CH_3$ | >130° (Z) |
| 81 | Cl | $-CONH-C_6H_4Cl$(4) | 188-193° |
| 82 | CN | $-CO-NH-CH_2-CH=CH_2$ | 150° (Z) |
| 83 | H | H | 167° |

sowie folgende Verbindungen der Formel

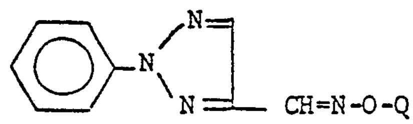

| Verb. Nr. | Q | Schmelzpunkt |
|---|---|---|
| 84 | H | 135-136° |
| 85 | $-CONHCH_3$ | 130° |
| 86 | $-CONH-C_3H_7(n)$ | 118° |
| 87 | $-CONH-CH_2CH_2-Cl$ | 167-171° |
| 88 | $-CON(CH_3)_2$ | 120-128° |

sowie folgende Verbindungen der Formel

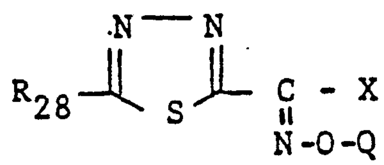

| Verb. Nr. | X | $R_{28}$ | Q | Schmelzpunkt |
|---|---|---|---|---|
| 89 | CN | $CH_3-S-$ | H | > 200°(Z) |
| 90 | CN | $C_2H_5-O-$ | H | 193°(Z) |
| 91 | CN | $CH_3-S-$ | $-CH_2-CN$ | 111° |
| 92 | CN | $CH_3-S-$ | $-CH(CH_3)COOCH_3$ | 96° |
| 93 | CN | $C_2H_5-O-$ | $-CH_2-CN$ | (halbfest) |
| 94 | CN | $C_2H_5-O-$ | $-CH(CH_3)COOCH_3$ | 58-63° |
| 95 | Cl | $C_6H_5$ | H | 222°(Z) |
| 96 | Cl | $CH_3-S-$ | H | 187° |
| 97 | Cl | $CH_3-SO_2-$ | H | 187°(Z) |
| 98 | $-COOC_2H_5$ | $CH_3O-$ | H | 151-156° |
| 99 | $-COOC_2H_5$ | $CH_3S-$ | H | 130°(Z) |
| 100 | $-COOC_2H_5$ | $CH_3SO-$ | H | 134°(Z) |
| 101 | $-COOCH_3$ | $CH_3S-$ | H | |
| 102 | $-COOCH_3$ | $CH_3SO$ | H | 138°(Z) |

sowie folgende Verbindungen der Formel

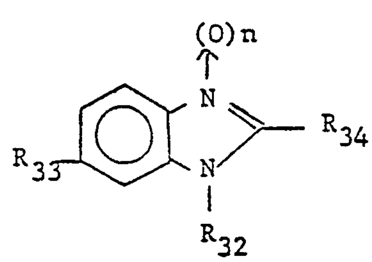

| Verb. Nr. | n | $R_{32}$ | $R_{33}$ | $R_{34}$ | Schmelz-punkt |
|---|---|---|---|---|---|
| 103 | 1 | $-CH_3$ | H | -CH=NOH | 228°(Z) |
| 104 | 1 | $-CH_3$ | Cl | -CH=NOH | Smp.145-7° |
| 105 | 1 | $-C_6H_5$ | H | -CH=NOH | Smp.208-209° |
| 106 | 0 | $-COOCH_3$ | H | -C(Cl)=NOH | 166° (Z) |

sowie folgende Verbindungen der Formel

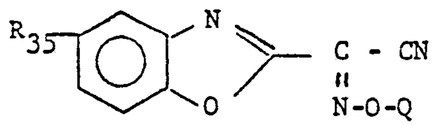

| Verb. Nr. | $R_{35}$ | Q | Schmelz-punkt |
|---|---|---|---|
| 107 | tert.$C_4H_9$ | H | 101-105° |
| 108 | tert.$C_4H_9$ | $-CON(CH_3)_2$ | 190° |
| 109 | $CH_3$ | H | 204° |
| 110 | $CH_3$ | $-CON(CH_3)_2$ | 194° |
| 111 | Cl | H | 208° |
| 112 | Cl | $-CON(CH_3)_2$ | 177° |
| 113 | $CH_3$ | $-CH_2-CN$ | |
| 114 | $CH_3$ | $-CH_2-CH=CH-CH_3$ | |

sowie folgende Verbindungen der Formel

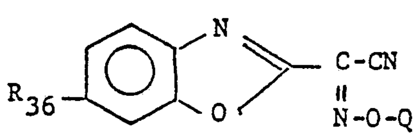

| Verb. Nr. | $R_{36}$ | Q | Schmelzpunkt |
|---|---|---|---|
| 115 | $CH_3$ | H | 223° |
| 116 | $CH_3$ | $-CON(CH_3)_2$ | 211° |
| 117 | $NO_2$ | H | 122-124° (Z) |
| 118 | $NO_2$ | $-CON(CH_3)_2$ | ca. 180° |
| 119 | $CH_3$ | $-CH_2CN$ | fest |
| 120 | $CH_3$ | $-CH_2-CH=CH_2$ | fest |
| 121 | $CH_3$ | $-CH_2-C\equiv CH$ | fest |

Wie schon erwähnt, kommen für die Verwendung der Verbindungen der Formel I zum Schutze von Kulturpflanzen vor Agrarchemikalien verschiedene Methoden und Techniken in Betracht :

1. Samenbeizung

a) Beizung der Samen mit einem als Spritzpulver formulierten Wirkstoff durch Schütteln in einem Gefäss bis zur gleichmässigen Verteilung auf der Samenoberfläche (Trockenbeizung). Man verwendet dabei etwa 10 bis 500 g Wirkstoff der Formel I (40 g bis 2 kg Spritzpulver) pro 100 kg Saatgut.

b) Beizung der Samen mit einem Emulsionskonzentrat des Wirkstoffs der Formel I nach der Methode a) (Nassbeizung).

c) Beizung durch Tauchen des Saatguts in eine Brühe mit 50-3 200 ppm Wirkstoff der Formel I während 1-72 Stunden und gegebenenfalls nachfolgendes Trocknen der Samen (Tauchbeizung).

Die Beizung des Saatguts oder die Behandlung des angekeimten Sämlings sind naturgemäss die bevorzugten Methoden der Applikation, weil die Wirkstoffbehandlung vollständig auf die Zielkultur gerichtet ist. Man verwendet in der Regel 10 g bis 500 g, vorzugsweise 50 bis 250 g AS pro 100 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

2. Applikation aus Tankmischung

Eine flüssige Aufarbeitung eines Gemisches von Gegenmittel und Herbizid (gegenseitiges Mengenverhältnis zwischen 1 : 20 und 5 : 1) wird verwendet, wobei die Aufwandmenge an Herbizid 0,1 bis 6 kg pro Hektar beträgt. Solche Tankmischung wird vorzugsweise vor oder unmittelbar nach der Aussaat appliziert oder 5-10 cm tief in den noch nicht gesäten Boden eingearbeitet.

3. Applikation in die Saatfurche

Das Gegenmittel wird als Emulsionskonzentrat, Spritzpulver oder als Granulat in die offene besäte Saatfurche eingebracht und hierauf wird nach dem Decken der Saatfurche in normaler Weise das Herbizid im Vorauflaufverfahren appliziert.

Prinzipiell kann das Gegenmittel also vor, zusammen mit oder nach dem Pestizid appliziert werden, und seine Anwendung kann auf die Samen oder auf das Feld vor oder nach dem Säen oder in gewissen Fällen auch nach dem Auflaufen der Saat erfolgen.

4. Kontrollierte Wirkstoffabgabe

Der Wirkstoff wird in Lösung auf mineralische Granulatträger oder polymerisierte Granulate (Harnstoff/Formaldehyd) aufgezogen und trocknen gelassen. Gegebenenfalls kann ein Ueberzug aufgebracht werden (Umhüllungsgranulate) der es erlaubt, den Wirkstoff über einen bestimmten Zeitraum dosiert abzugeben.

Selbstverständlich können auch alle sonstigen Methoden der Wirkstoffapplikation angewendet werden. Dafür werden im folgenden Beispiele gegeben.

Die Verbindungen der Formel I können für sich allein oder zusammen mit geeigneten Trägern und/oder anderen Zuschlagstoffen verwendet werden. Geeignete Träger und Zuschlagstoffe können fest oder flüssig sein und entsprechen den in der Formulierungstechnik üblichen Stoffen wie z. B. natürlichen oder regenerierten mineralischen Stoffen, Lösungs-, Dispergier-, Netz-, Haft-, Verdickungs-, Binde- oder Düngemitteln.

Der Gehalt an Wirkstoff in handelsfähigen Mitteln liegt zwischen 0,1 bis 90 %.

Zur Applikation können die Verbindungen der Formel I in den folgenden Aufarbeitungsformen vorliegen (wobei die Gewichts-Prozentangaben in Klammern vorteilhafte Mengen an Wirkstoff darstellen) :

Feste Aufarbeitungsformen : Stäubemittel und Streumittel (bis zu 10 %) Granulate, Umhüllungsgranulate, Imprägnierungsgranulate und Homogengranulate, Pellets (Körner) 1 bis 80 %) ;

Flüssige Aufarbeitungsformen :

a) in Wasser dispergierbare Wirkstoffkonzentrate : Spritzpulver (wettable powders) und Pasten (25-90 % in der Handelspackung, 0,01 bis 15 % in gebrauchsfertiger Lösung) ; Emulsions- und Lösungskonzentrate (10 bis 50 % ; 0,01 bis 15 % in gebrauchsfertiger Lösung) ;

b) Lösungen (0,1 bis 20 %) ; Aerosole

Die Wirkstoffe der Formel I vorliegender Erfindung können beispielsweise wie folgt formuliert werden :

Stäubemittel : Zur Herstellung eines a) 5 %igen und b) 2 %igen Stäubemittels werden die folgenden Stoffe verwendet :

a) 5 Teile Wirkstoff
95 Teile Talkum ;

b) 2 Teile Wirkstoff
1 Teil hochdisperse Kieselsäure,
97 Teile Talkum ;

Die Wirkstoffe werden mit den Trägerstoffen vermischt und vermahlen und können in dieser Form zur Anwendung verstäubt werden.

Granulat : Zur Herstellung eines 5 %igen Granulates werden die folgenden Stoffe verwendet :

5 Teile Wirkstoff
0,25 Teile Epichlorhydrin,
0,25 Teile Cetylpolyglykoläther,
3,50 Teile Polyäthylenglykol
91 Teile Kaolin (Korngrösse 0,3-0,8 mm).

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht, und anschliessend wird das Aceton im Vakuum verdampft. Ein derartiges Mikrogranulat lässt sich vorteilhaft in Saatfurchen einarbeiten.

Spritzpulver : Zur Herstellung eines a) 70 %igen b) 40 %igen c) und d) 25 %igen e) 10 %igen Spritzpulvers werden folgende Bestandteile verwendet :

a) 70 Teile Wirkstoff
5 Teile Natriumdibutylnaphtylsulfonat,
3 Teile Naphtalinsulfonsäuren-Phenolsulfonsäuren-Formaldehyd-Kondensat 3 : 2 : 1,
10 Teile Kaolin,
12 Teile Champagne-Kreide ;

b) 40 Teile Wirkstoff
5 Teile Ligninsulfonsäure-Natriumsalz,
1 Teil Dibutylnaphtalinsulfonsäure-Natriumsalz,
54 Teile Kieselsäure ;

c) 25 Teile Wirkstoff
4,5 Teile Calcium-Ligninsulfonat,
1,9 Teile Champagne-Kreide/Hydroxyäthylcellulose-Gemisch (1 : 1),
1,5 Teile Natrium-dibutyl-naphthalinsulfonat,
19,5 Teile Kieselsäure,
19,5 Teile Champagne-Kreide,
28,1 Teile Kaolin ;

d) 25 Teile Wirkstoff
2,5 Teile Isooctylphenoxy-polyoxyäthylen-äthanol,
1,7 Teile Champagne-Kreide/Hydroxyäthylcellulose-Gemisch (1 : 1),
8,3 Teile Natriumaluminiumsilikat,
16,5 Teile Kieselgur,
46 Teile Kaolin ;

e) 10 Tiele Wirkstoff
3 Teile Gemisch der natriumsalze von gesättigten Fettalkoholsulfaten,
5 Teile Naphthalinsulfonsäure/Formaldehyd-Kondensat,
82 Teile Kaolin ;

Die Wirkstoffe werden in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Saatbeizung und Tauchbehandlung von Stecklingen verwenden lassen.

Emulgierbare Konzentrate : Zur Herstellung eines 25 %igen emulgierbaren Konzentrates werden folgende Stoffe verwendet :

25 Teile Wirkstoff
2,5 Teile epoxydiertes Pflanzenöl,
10 Teile eines Alkylarylsulfonat/Fettalkoholpolyglykoläther-Gemisches,
5 Teile Dimethylformamid,
57,5 Teile Xylol.

Aus solchen Konzentration können durch Verdünnen mit Wasser Emulsionen jeder gewünschten Konzentration hergestellt werden, die besonders zur Saatbeizung und Tauchbehandlung von Jungpflanzen geeignet sind.

Vorauflauf-Antidote-Test (Grundtest)

Allgemeine Methodik:

Kleine Blumentöpfe (oberer ∅ 6 cm) werden mit Gartenerde gefüllt, in die Pflanzenkultur eingesät, bedeckt und leicht festgedrückt wird. Dann wird die als Antidote zu prüfende Substanz als (aus einem Spritzpulver erhaltene) verdünnte Lösung in einer Menge aufgesprüht, die 4 kg AS/ha entspricht. Unmittelbar danach wird in entsprechender Weise das Herbizid aufgesprüht. Nach 18 Tagen Stehen bei ca. 20-23 °C und 60-70 % relativer Luftfeuchtigkeit wird nach einer linearen Skala von 1 bis 9 ausgewertet, wobei 1 totale Pflanzenschädigung und 9 unbeeinträchtigter Gesundheitszustand bedeuten. Als Kontrolle dienen Pflanzen ohne Antidote-Schutz.

Es wurden verwendet:

1. 1,5 kg AS/ha α-[4-(p-Trifluormethylphenoxy)-phenoxy]-propionsäure-n-butoxyäthylester in Mais der Sorte « Orla 264 ».
2. 1,5 kg AS/ha Metolachlor = N-(1-Methyl-2-methoxyäthyl)-N-chloracetyl-2-äthyl-6-methylanilin in Sorghum-Hirse der Sorte « Funk G-522 ».
3. 2,0 kg AS/ha Prometryn = 2,4-bis(Isopropylamino)-6-methylthio-s-triazin in Soja.
4. 2,0 kg AS/ha 4-Aethylamino-6-tert.butylamino-2-chlor-s-triazin in Weizen der Sorte « Farnese ».
5. 4,0 kg AS/ha Prometryn = 2,4-bis(Isopropylamino)-6-methylthio-s-triazin in Sorghum-Hirse der Sorte « Funk G-522 ».
6. 2,0 kg AS/ha α-[4-(p-Trifluormethylphenoxy)-phenoxy]-propionsäure-n-butoxyäthylester in Gerste der Sorte « Mazurka ».

Verbindungen der Formel I erzielen in diesen Versuchen gute Antidote-Wirkung. Als Beispiele seien die folgenden Ergebnisse genannt.

| Testvariante | Verb. Nr. | Note des Herbizideinflusses (ohne/mit Antidote) |
|---|---|---|
| 5 | 104 | 6/8 |
| 6 | 94 | 6/8 |
| 5 | 26 | 3/6 |
| 5 | 40 | 1/6 |
| 5 | 84 | 1/6 |
| 5 | 104 | 6/8 |
| 5 | 65 | 1/5 |

Antidote-Wirkung bei getrennter Applikation (Antidote-Vorauflauf. Herbizid-Nachauflauf)

Allgemeine Methodik:

Kleine Blumentöpfe (oberer ∅ 6 cm) werden mit sandiger Lehmerde gefüllt, in die die Kulturpflanze eingesät wird. Nach dem Bedecken des Samens sprüht man die als Antidote zu prüfende Substanz in verdünnter Lösung und in einer Menge auf die Oberfläche, die umgerechnet 4 kg AS/ha beträgt. Man hält bei 20-23 °C und 60-70 % relativer Luftfeuchtigkeit. Wenn die Pflanzen nach 10 Tagen das 2- bis 3-Blattstadium erreicht haben, werden sie, wie nachfolgend angegeben, mit der entsprechenden Menge Herbizid behandelt. 14 Tage nach Herbizid-Applikation wird nach einer linearen Skala von 1 bis 9 ausgewertet, wobei 1 totale Pflanzenschädigung und 9 unbeeinträchtigter Gesundheitszustand bedeuten. Als Kontrolle dienen Pflanzen ohne Antidote-Schutz.

Man verwendet:

1. 4,0 kg AS/ha Ametryn = 2-Aethylamino-4-isopropylamino-6-methylthio-s-triazin in Mais der Sorte « Orla 264 ».
2. 1,0 kg AS/ha Prometryn = 2,4-bis (Isopropylamino)-6-methylthio-s-triazin in Sorghum-Hirse der Sorte « Funk G-522 ».

3. 0,25 kg AS/ha α-[4-(p-Trifluormethylphenoxy)-phenoxy]-propionsäure-n-butoxyäthylester in Gerste der Sorte « Mazurka ».

Verbindungen der Formel I zeigten in diesen Versuchen gute Antidote-Wirkung.

Antidote-Wirkung an verpflanztem Reis bei getrennter Applikation (Antidote-Vorauflauf. Herbizid-Nachauflauf)

Plastiktöpfe (8 × 8 cm, 10 cm Höhe) werden bis 2 cm unter den Rand mit Erde im sumpfig-nassen Zustand gefüllt. Die als Antidote zu prüfende Substanz wird in verdünnter Lösung und in einer Menge auf die Oberfläche gesprüht, die 4 kg AS/ha entspricht. Reispflanzen der Sorte « IR-8 » werden im 1 1/2- bis 2-Blattstadium in die so vorbereiteten Töpfe verpflanzt. Am nächsten Tag wird der Wasserstand auf ca. 1,5 cm erhöht. 4 Tage nach Verpflänzung werden umgerechnet 0,75 kg AS/ha von 2-Aethylamino-4-(1,2-dimethyl-n-propylamino)-6-methylthio-s-triazin in Granulatform ins Wasser gegeben. Die Temperatur beträgt während der Versuchsdauer 26-28 °C, die relative Luftfeuchtigkeit 60-80 %. 20 Tage nach Herbizidbehandlung wird nach einer linearen Skala von 1 bis 9 ausgewertet, wobei 1 totale Pflanzenschädigung und 9 unbeeinträchtigter Gesundheitszustand bedeuten. Als Kontrolle dienen Pflanzen ohne Antidote-Schutz.

Mit den Verbindungen der Formel I wurden gute Antidote-Resultate erzielt. Als Beispiele seien die folgenden Ergebnisse genannt.

| Verb. Nr. | Note des Herbizideinflusses (ohne/mit Antidote) |
|---|---|
| 115 | 5/7 |
| 105 | 3/8 |
| 104 | 3/5 |
| 26 | 4/6 |
| 69 | 5/7 |
| 16 | 5/8 |
| 33 | 5/7 |

Vorauflauf-Antidote-Test in Nährlösung

Es wird eine Hewitt-Nährlösung hergestellt, die die nachstehend angegebene Menge Herbizid sowie 10 ppm des zu prüfenden Antidotes enthält.

Man verwendet Kultursamen, der in der angegebenen Prüfkonzentration von dem eingesetzten Herbizid erwartungsgemäss geschädigt werden sollte und sät ihn in gekörntes Zonolith (= expandiertes Vermikulit), das sich in einem an der Unterseite durchlöcherten Plastik-Blumentopf (oberer ∅ 6 cm) befindet. Dieser wird in einen zweiten durchsichtigen Plastik-Blumentopf (oberer ∅ 7 cm) gestellt, in dem sich sich ca. 50 ml der mit Herbizid und Antidote vorbereiteten Nährlösung befinden, die nunmehr kapillar im Füllmaterial des kleineren Topfes aufsteigt und Samen und keimende Pflanze benetzt. Täglich wird der Flüssigkeitsverlust mit reiner Hewitt-Nährlösung auf 50 ml ergänzt. 3 Wochen nach Testbeginn wird nach einer linearen Skala von 1 bis 9 ausgewertet, wobei 1 totale Pflanzenschädigung und 9 unbeeinträchtigter Gesundheitszustand bedeuten. Die parallel angewandte Kontroll-Lösung enthält keinen Antidote-Zusatz.

Man verwendet :

1. 4 ppm Prometryn = 2,4-bis (Isopropylamino)-6-methylthio-s-triazin in Sorghum-Hirse der Sorte « Funk G-522 ».

2. 4 ppm 4-Aethylamino-6-tert.butylamino-2-chlor-s-triazin in Weizen der Sorte « Farnese ».

3. 4 ppm α-[4-(p-Trifluormethylphenoxy)-phenoxy]-propionsäure-n-butoxyäthylester in Gerste der Sorte « Mazurka ».

4. 5 ppm Metolachlor = N-(1-Methyl-2-methoxy-äthyl)-N-chloracetyl-2-äthyl-6-methylanilin in Sorghum-Hirse der Sorte « Funk G-522 ».

Mit den Verbindungen der Formel I erhält man gute Antidote Wirkung. Als Beispiele seien genannt :

| Testvariante | Verb. Nr. | Note des Herbizideinflusses (ohne/mit Antidote) |
|---|---|---|
| 4 | 96 | 5/8 |
| 1 | 48 | 2/5 |
| 3 | 51 | 2/7 |
| 3 | 54 | 3/8 |
| 3 | 49 | 5/7 |

Vorauflauf-Antidote Test in Nährlösung (Reis)

Es wird eine Hewitt-Nährlösung hergestellt, die zusätzlich 10 ppm des zu prüfenden Antidotes enthält.

Reissamen der Sorte « IR-8 » wird in inertes Füllmaterial (gekörntes Zonolith) gesät, das sich in einem an der Unterseite durchlöcherten Plastik-Blumentopf (oberer ∅ 6 cm) befindet. Dieser wird in einen zweiten durchsichtigen Plastik-Blumentopf (oberer ∅ 7 cm) gestellt, in dem sich ca. 50 ml der vorbereiteten Nährlösung befinden, die nunmehr kapillar im Füllmaterial des kleineren Topfes aufsteigt und Samen und keimende Pflanze benetzt. Täglich wird der Flüssigkeitsverlust mit reiner Hewit-Nährlösung auf 50 ml ergänzt. Nach 15 Tagen werden die Reispflanzen im 2- bis 2 1/2-Blattstadium in rechteckige Plastiktöpfe (8 × 8 cm, 10 cm Höhe) verpflanzt, die mit 500 ml sumpfignasser Erde gefüllt sind. Am nächsten Tag wird der Wasserstand darin auf 1-2 cm über Boden-Niveau erhöht. 4 Tage nach Verpflanzung gibt man das Herbizid 2-Aethylamino-4-(1,2-dimethyl-n-propylamino)-6-methylthio-s-triazin in Granulatform und in einer Menge zu, die umgerechnet 0,75 kg AS/ha beträgt. 3 Wochen nach Herbizidzugabe wird nach einer linearen Skala von 1 bis 9 ausgewertet, wobei 1 totale Pflanzenschädigung und 9 unbeeinträchtigter Gesundheitszustand bedeuten. Die parallel angewandte Kontroll-Lösung enthält keinen Antidote-Zusatz. Verbindungen der Formel I zeigten Antidote-Wirkung in diesem Versuch.

Nachauflauf-Antidote-Test in Nährlösung

Allgemeine Methodik :

Man füllt kleine Plastik-Blumentöpfe (oberer ∅ 6 cm), die an der Unterseite durchlöchert sind, mit gekörntem Zonolith und sät den Kultursamen ein. Dann wird der Topf in einen zweiten durchsichtigen Plastik-Blumentopf (oberer ∅ 7 cm) gestellt, in dem sich 50 ml Wasser befinden, das kapillar aufsteigt und den Samen benetzt. Ab 5. Tag wird der laufende Wasserverlust mit Hewitt-Nährlösung ausgeglichen. Ab 15. Tag, wenn sich die Kulturpflanze im 1 1/2-bis 2-Blattstadium befindet, wird in die wieder auf 50 ml ergänzte Nährlösung 10 ppm des zu prüfenden Antidotes + die unten angegebene Menge Herbizid zugegeben. Ab 16. Tag wird der Flüssigkeitsverlust wieder durch reine Hewitt-Nährlösung ausgeglichen. Während der gesamten Testdauer beträgt die Temperatur 20-23 °C bei einer relativen Luftfeuchtigkeit von 60-70 %. 3 Wochen nach Zugabe des Herbizids und des Antidotes erfolgt die Auswertung nach einer linearen Skala von 1 bis 9, wobei 1 totale Pflanzenschädigung und 9 unbeeinträchtigter Gesundheitszustand bedeuten.

Testvarianten :

1. 15 ppm α-[4-(3,5-Dichlorpyridyl-2-oxy)-phenoxy]-propionsäure-propargylthioloester in Weizen der Sorte « Zenith ».
2. 4 ppm 4-Aethylamino-6-tert.butylamino-2-chlor-s-triazin in Weizen der Sorte « Zenith ».
3. 2 ppm α-[4-p-Trifluormethylphenoxy)-phenoxy]-propionsäure-n-butoxyäthylester in Mais der Sorte « Orla ».
4. 8 ppm α-[4-(p-Trifluormethylphenoxy)-phenoxy]-propionsäure-n-butoxyäthylester in Sorghum-Hirse der Sorte « Funk G-522 ».
5. 4 ppm Prometryn = 2,4-bis(Isopropylamino)-6-methylthio-s-triazin in Sorghum-Hirse der Sorte « Funk G-522 ».
6. 8 ppm α-[4-(3,5-Dichlorpyridyl-2-oxy)-phenoxy]-propionsäuremethylester in Weizen der Sorte « Zenith ».

Verbindungen der Formel I erzielen bei diesen Versuchen gute Antidote-Wirkung. Als Beispiele seien die folgenden Ergebnisse genannt :

| Testvariante | Verb. Nr. | Note des Herbizideinflusses (ohne/mit Antidote) |
|---|---|---|
| 1 | 94 | 3/5 |
| 5 | 10 | 2/4 |
| 1 | 112 | 3/7 |
| 1 | 92 | 6/8 |

Antidote Test Samenquellung (Seed Soaking)

Reissamen der Sorte IR 8 werden während 48 Stunden mit Lösungen der Testsubstanzen von 10 oder 100 ppm getränkt. Anschliessend werden die Samen etwa 2 Stunden trocknen gelassen, bis sie nicht mehr kleben. Rechteckige Plastiktöpfe (8 × 8 cm, 10 cm Höhe) werden bis 2 cm unter den Rand mit

sandigem Lehm gefüllt. 4 g Samen werden pro Topf gesät und nur ganz schwach gedeckt (etwa Durchmesser des Samenkorns). Die Erde wird in einem feuchten (nicht sumpfigen) Zustand gehalten. Dann werden wahlweise das Herbizid N-(1-Methyl-2-methoxyäthyl)-N-chloracetyl-2-äthyl-6-methylanilin oder N-Propoxyäthyl-N-chloracetyl-2,6-diäthylanilin in verdünnter Lösung und in einer Menge appliziert, die umgerechnet 1,5 kg AS/ha entspricht. 18 Tage nach dem Verpflanzen wird nach einer linearen Skala von 1 bis 9 ausgewertet, wobei 1 totale Pflanzenschädigung und 9 unbeeinträchtigter Gesundheitszustand bedeuten.

Verbindungen der Formel I zeigen in diesem Versuch gute Antidote-Wirkung. Als Beispiele seien die folgenden Resultate genannt.

| | Verb. Nr. | Note des Herbizideinflusses (ohne/mit Antidote) |
|---|---|---|
| 10 ppm | 5 | 2/4 |
| 10 ppm | 48 | 2/5 |
| 100 ppm | 51 | 2/4 |
| 10 ppm | 53 | 3/7 |
| 100 ppm | 53 | 3/6 |
| 10 ppm | 32 | 3/6 |
| 10 ppm | 50 | 3/6 |
| 10 ppm | 84 | 2/6 |
| 100 ppm | 34 | 3/6 |
| 100 ppm | 36 | 3/6 |
| 100 ppm | 38 | 3/7 |

**Ansprüche** (für die Vertragsstaaten : BE, CH, DE, FR, GB, IT, NL)

1. Verwendung von Verbindungen der Formel I

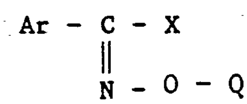

(I)

zum Schutz von Pflanzen Kulturen vor der phytotoxischen Wirkung von aggressiven Agrarchemkalien, in welcher Formel

Ar einen fünfgliedrigen, ungesättigten, gegebenenfalls benzokondensierten heterocyclischen Ring, welcher 2 oder 3 Heteroatome aus der Gruppe O, S und N, von denen mindestens eines ein Stickstoffatom ist, bedeutet, der am heterocyclischen oder aromatischen Teil durch $R_1$, $R_2$ und $R_3$ substituiert ist und/oder gegebenenfalls durch Oxo oder Thioxo substituiert ist, wobei $R_1$ für Wasserstoff, Halogen, $C_1$-$C_4$-Alkyl oder $C_1$-$C_4$-Alkoxy, $R_2$ für Wasserstoff, Halogen, Nitro, $C_1$-$C_4$-Alkyl, Halogen-$C_1$-$C_4$-Alkyl oder $C_1$-$C_4$-Alkoxy und $R_3$ für Wasserstoff oder Phenyl stehen oder, wenn als Heterocyclus ein Thiadiazol vorliegt, $R_1$ und $R_2$ für Wasserstoff und $R_3$ für $C_1$-$C_4$-Alkoxy, $C_1$-$C_4$-Alkylthio, $C_1$-$C_4$-Alkylsulfinyl, $C_1$-$C_4$-Alkylsulfonyl oder Phenyl stehen,

X Wasserstoff, Cyan, Halogen, $C_1$-$C_4$-Alkyl oder $C_1$-$C_4$-Alkoxycarbonyl sind und

Q Wasserstoff, ein Metallkation, $C_1$-$C_4$-Alkyl, welches gegebenenfalls durch Cyan oder Carbamoyl substituiert ist, $C_2$-$C_4$-Alkenyl, $C_2$-$C_4$-Alkinyl, $C_1$-$C_4$-Alkylcarbonyl, Halogen-$C_1$-$C_4$-Alkylcarbonyl, $C_2$-$C_4$-Alkenylcarbonyl, $C_1$-$C_4$-Alkoxycarbonyl, $C_1$-$C_4$-Alkylthio-carbonyl, $C_1$-$C_4$-Alkylsulfonyl, $C_1$-$C_4$-Alkoxycarbonyl-$C_1$-$C_4$-Alkyl, Furanoyl, Benzoyl, welches gegebenenfalls durch Substituenten der Gruppe Methyl, Methoxy, Halogen oder nitro ein- bis zweifach substituiert ist, Benzylcarbonyl, Aminocarbonyl, welches am Stickstoffatom gegebenenfalls ein- oder zweimal durch $C_1$-$C_4$-Alkyl, $C_2$-$C_4$-Alkenyl, Halogen-$C_1$-$C_4$-Alkyl oder $C_1$-$C_4$-Alkoxy oder einmal durch Phenyl, welches gegebenenfalls Trifluormethyl und/oder Halogen als Substituenten trägt, substituiert ist, oder Phenylsulfonyl bedeuten.

2. Verwendung gemäss Anspruch 1 einer Verbindung der Formel

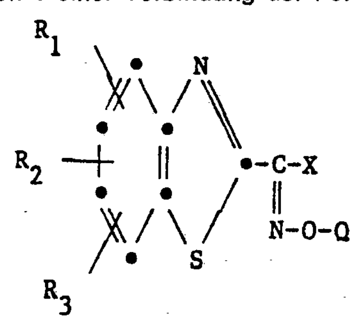

worin $R_1$, $R_2$ und $R_3$ die für Formel I angegebenen Bedeutungen haben, X Cyan oder $C_1$-$C_4$-Alkoxycarbonyl und Q $C_1$-$C_4$-Alkyl, welches gegebenenfalls durch Cyan substituiert ist, $C_2$-$C_4$-Alkenyl, $C_2$-$C_4$-Alkinyl, $C_1$-$C_4$-Alkylcarbonyl, $C_2$-$C_4$-Alkenylcarbonyl, $C_1$-$C_4$-Alkylsulfonyl, Benzoyl, welches gegebenenfalls durch Halogen substituiert ist, oder Aminocarbonyl, welches am Stickstoffatom gegebenenfalls ein- oder zweimal durch $C_1$-$C_4$-Alkyl oder $C_2$-$C_4$-Alkenyl oder einmal durch Phenyl, welches gegebenenfalls Trifluormethyl und/oder Halogen als Substituenten trägt, substituiert ist, bedeuten.

3. Verwendung gemäss Anspruch 2, worin bei der zu verwendenden Verbindung $R_1$, $R_2$ und $R_3$ Wasserstoff bedeuten.

4. Verwendung gemäss Anspruch 3 einer Verbindung der Formel

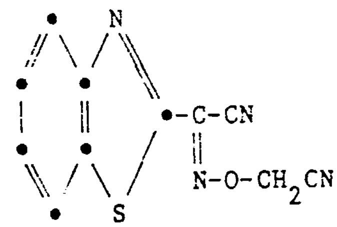

5. Verwendung gemäss Anspruch 1 einer Verbindung der Formel

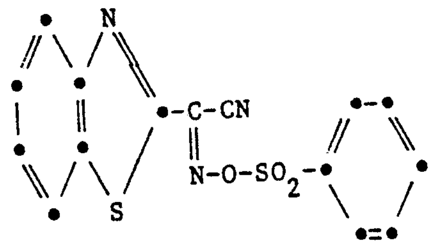

6. Verwendung gemäss Anspruch 1, einer Verbindung der Formel

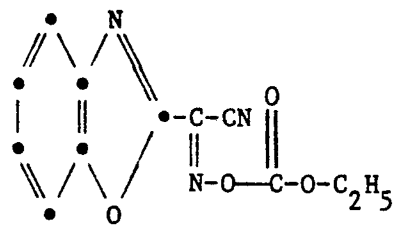

7. Verwendung gemäss Anspruch 1, einer Verbindung der Formel

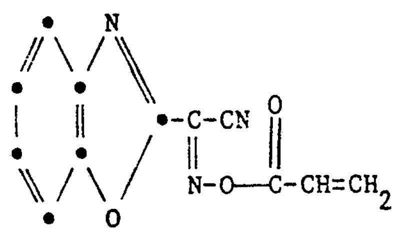

8. Verwendung gemäss Anspruch 1 einer Verbindung der Formel

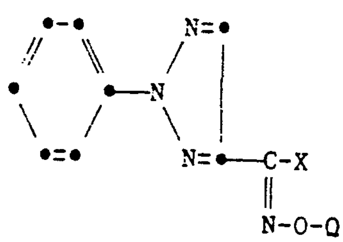

worin X Wasserstoff und Q Wasserstoff oder Aminocarbonyl, welches am Stickstoffatom durch $C_1$-$C_4$-Alkyl oder Halogen-$C_1$-$C_4$-Alkyl mono- oder disubstituiert ist, bedeuten.

9. Verwendung gemäss Anspruch 8 einer Verbindung der Formel

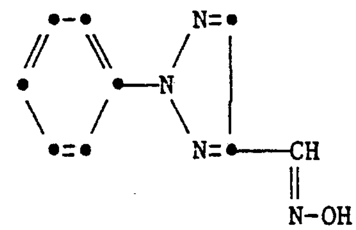

10. Verwendung gemäss Anspruch 1 einer Verbindung der Formel

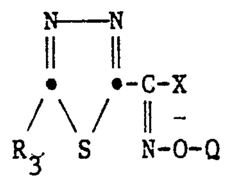

worin $R_3$ $C_1$-$C_4$-Alkoxy, $C_1$-$C_4$-Alkylthio, $C_1$-$C_4$-Alkylsulfinyl, $C_1$-$C_4$-Alkylsulfonyl oder Phenyl, X Wasserstoff, Cyan, Halogen, $C_1$-$C_4$-Alkyl oder $C_1$-$C_4$-Alkoxycarbonyl und Q Wasserstoff, oder $C_1$-$C_4$-Alkyl, welches gegebenenfalls durch Cyan substituiert ist, bedeuten.

11. Verwendung gemäss Anspruch 10, worin bei der zu verwendenden Verbindung X Cyan, Halogen oder $C_1$-$C_4$-Alkoxycarbonyl bedeutet.

12. Verwendung gemäss Anspruch 10 einer Verbindung der Formel

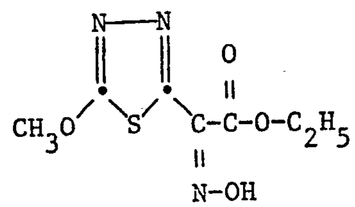

13. Verwendung gemäss Anspruch 1 einer Verbindung der Formel

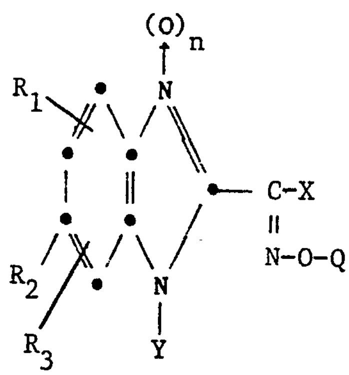

worin n die Zahl 0 oder 1, $R_1$ und $R_3$ Wasserstoff, $R_2$ Wasserstoff oder Halogen, Y, $C_1$-$C_4$-Alkyl, $C_1$-$C_4$-Alkoxycarbonyl oder Phenyl, X Wasserstoff, Cyan, Halogen oder $C_1$-$C_4$-Alkyl und Q Wasserstoff, $C_1$-$C_4$-Alkylcarbonyl, Benzoyl, welches gegebenenfalls durch Halogen substituiert ist, oder Aminocarbonyl, welches am Stickstoffatom gegebenenfalls ein- oder zweimal durch $C_1$-$C_4$-Alkyl, oder einmal durch Phenyl, welches gegebenenfalls Halogen als Substituenten trägt, substituiert ist, bedeuten.

14. Verwendung gemäss Anspruch 13 einer Verbindung der Formel

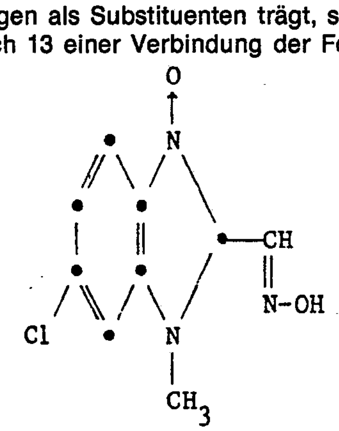

15. Verwendung gemäss Anspruch 13 einer Verbindung der Formel

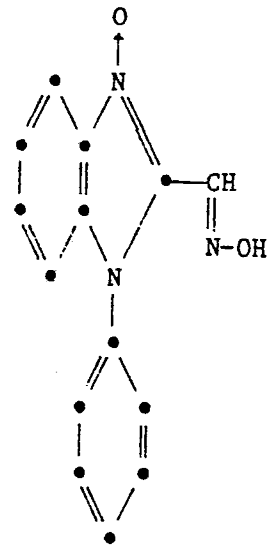

16. Verwendung gemäss Anspruch 1 einer Verbindung der Formel

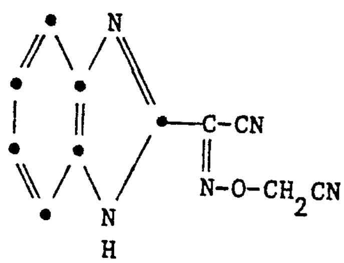

17. Verwendung gemäss Anspruch 13 einer Verbindung der Formel

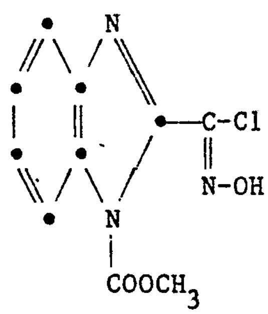

18. Mittel zum Schützen von Kulturpflanzen vor aggressiven Agrarchemikalien enthaltend als Antidote ein Oximderivat der Formel I wie im Anspruch 1 definiert, zusammen mit einem geeigneten Trägermaterial.

19. Antagonistisch wirkende Mittel zum Schützen von Kulturpflanzen vor aggressiven Agrarchemikalien enthaltend als Wirkstoffe a) die Agrarchemikalien sowie b) ein für Kulturpflanzen dazu als Antidote wirkendes Oximderivat der Formel I wie im Anspruch 1 definiert im Mischungsverhältnis 100 : 1 bis 1 : 5, vorzugsweise 20 : 1 bis 1 : 1.

20. Mittel gemäss Anspruch 19, worin die Agrarchemikalie ein Herbizid ist.

21. Vermehrungsgut von Kulturpflanzen, behandelt mit einem Oximderivat der Formel I wie im Anspruch 1 definiert.

22. Vermehrungsgut gemäss Anspruch 22, dadurch gekennzeichnet, dass es sich um Saatgut handelt.

23. Oximderivate der Formel I wie im Anspruch 1 definiert.

24. Ein Oximderivat, wie in einem der Ansprüche 2 bis 17 definiert.

25. Ein Oximderivat gemäss Anspruch 23 aus der nachfolgenden Gruppe :

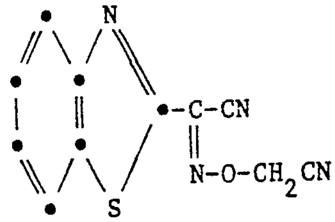

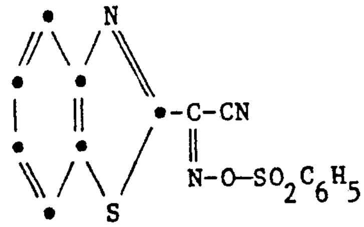

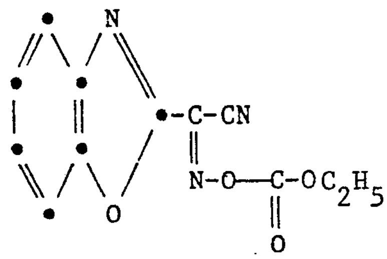

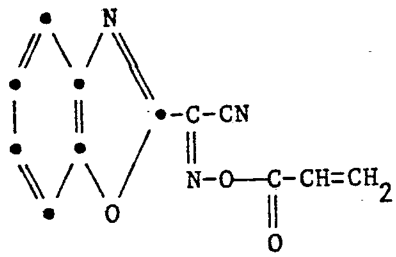

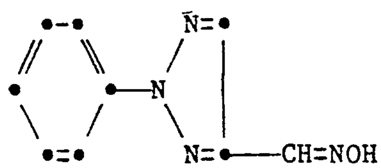

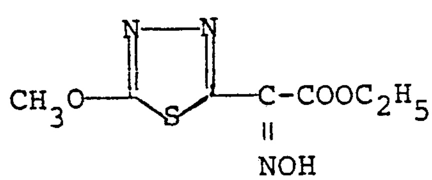

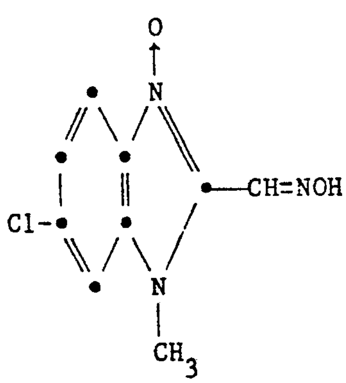

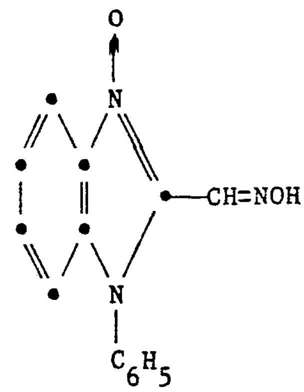

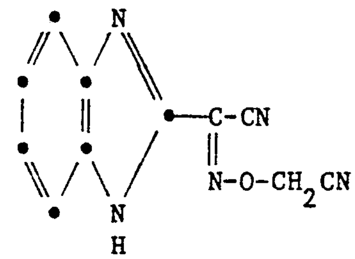

und

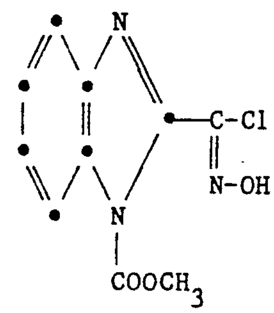

**Ansprüche** (für den Vertragsstaat AT)

1. Verwendung von Verbindungen der Formel I

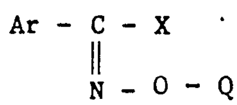

(I)

zum Schutz von Pflanzenkulturen vor der phytotoxischen Wirkung von aggressiven Agrarchemikalien, in welcher Formel

Ar einen fünfgliedrigen, ungesättigten, gegebenenfalls benzokondensierten heterocyclischen Ring, welcher 2 oder 3 Heteroatome aus der Gruppe O, S und N, von denen mindestens eines ein Stickstoffatom ist, bedeutet, der am heterocyclischen oder aromatischen Teil durch $R_1$, $R_2$ und $R_3$ substituiert ist und/oder gegebenenfalls durch Oxo oder Thioxo substituiert ist, wobei $R_1$ für Wasserstoff, Halogen, $C_1$-$C_4$-Alkyl oder $C_1$-$C_4$-Alkoxy, $R_2$ für Wasserstoff, Halogen, Nitro, $C_1$-$C_4$-Alkyl, Halogen-$C_1$-$C_4$-Alkyl oder $C_1$-$C_4$-Alkoxy und $R_3$ für Wasserstoff oder Phenyl stehen oder, wenn als Heterocyclus ein Thiadiazol vorliegt, $R_1$ und $R_2$ für Wasserstoff und $R_3$ für $C_1$-$C_4$-Alkoxy, $C_1$-$C_4$-Alkylthio, $C_1$-$C_4$-Alkylsulfinyl, $C_1$-$C_4$-Alkylsulfonyl oder Phenyl stehen,

X Wasserstoff, Cyan, Halogen, $C_1$-$C_4$-Alkyl oder $C_1$-$C_4$-Alkoxycarbonyl sind und

Q Wasserstoff, ein Metallkation, $C_1$-$C_4$-Alkyl, welches gegebenenfalls durch Cyan oder Carbamoyl substituiert ist, $C_2$-$C_4$-Alkenyl, $C_2$-$C_4$-Alkinyl, $C_1$-$C_4$-Alkylcarbonyl, Halogen-$C_1$-$C_4$-Alkylcarbonyl, $C_2$-$C_4$-Akenylcarbonyl, $C_1$-$C_4$-Alkoxycarbonyl, $C_1$-$C_4$-Alkylthiocarbonyl, $C_1$-$C_4$-Alkylsulfonyl, $C_1$-$C_4$-Alkoxycarbonyl-$C_1$-$C_4$-Alkyl, Furanoyl, Benzoyl, welches gegebenenfalls durch Substituenten der Gruppe Methyl, Methoxy, Halogen oder Nitro ein- bis zweifach substituiert ist, Benzylcarbonyl, Aminocarbonyl, welches am Stickstoffatom gegebenenfalls ein- oder zweimal durch $C_1$-$C_4$-Alkyl, $C_2$-$C_4$-Alkenyl, Halogen-$C_1$-$C_4$-Alkyl oder $C_1$-$C_4$-Alkoxy oder einmal durch Phenyl, welches gegebenenfalls Trifluormethyl und/oder Halogen als Substituenten trägt, substituiert ist, oder Phenylsulfonyl bedeuten.

2. Verwendung gemäss Anspruch 1 einer Verbindung der Formel

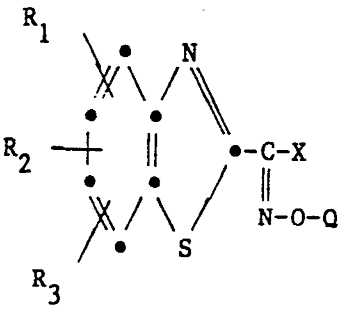

worin $R_1$, $R_2$ und $R_3$ die für Formel I angegebenen Bedeutungen haben, X Cyan oder $C_1$-$C_4$-Alkoxycarbonyl und Q $C_1$-$C_4$-Alkyl, welches gegebenenfalls durch Cyan substituiert ist, $C_2$-$C_4$-Alkenyl, $C_2$-$C_4$-Alkinyl, $C_1$-$C_4$-Alkylcarbonyl, $C_2$-$C_4$-Alkenylcarbonyl, $C_1$-$C_4$-Alkylsulfonyl, Benzoyl, welches gegebenenfalls durch Halogen substituiert ist, oder Aminocarbonyl, welches am Stickstoffatom gegebenenfalls ein- oder zweimal durch $C_1$-$C_4$-Alkyl oder $C_2$-$C_4$-Alkenyl oder einmal durch Phenyl, welches gegebenenfalls Trifluormethyl und/oder Halogen als Substituenten trägt, substituiert ist, bedeuten.

3. Verwendung gemäss Anspruch 2, worin bei der zu verwendenden Verbindung $R_1$, $R_2$ und $R_3$ Wasserstoff bedeuten.

4. Verwendung gemäss Anspruch 3 einer Verbindung der Formel

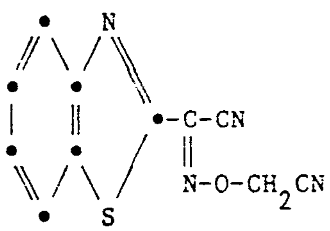

5. Verwendung gemäss Anspruch 1 einer Verbindung der Formel

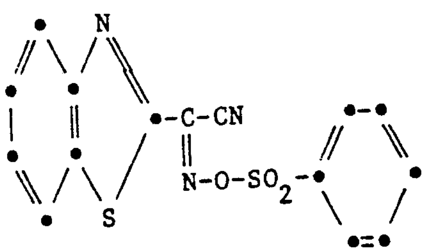

6. Verwendung gemäss Anspruch 1 einer Verbindung der Formel

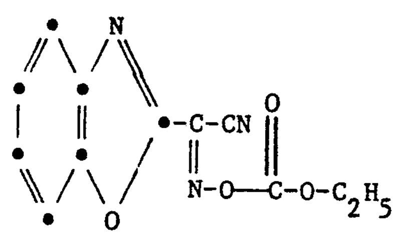

7. Verwendung gemäss Anspruch 1 einer Verbindung der Formel

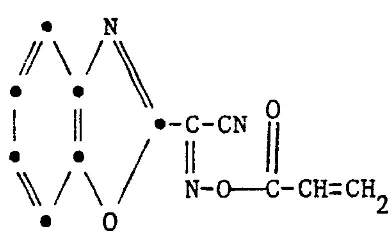

8. Verwendung gemäss Anspruch 1 einer Verbindung der Formel

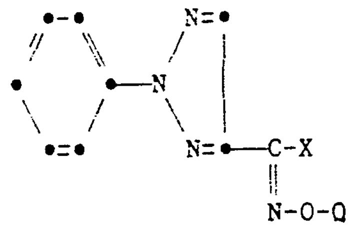

worin X Wasserstoff und Q Wasserstoff oder Aminocarbonyl, welches am Stickstoffatom durch $C_1$-$C_4$-Alkyl oder Halogen-$C_1$-$C_4$-Alkyl mono- oder disubstituiert ist, bedeuten.

9. Verwendung gemäss Anspruch 8 einer Verbindung der Formel

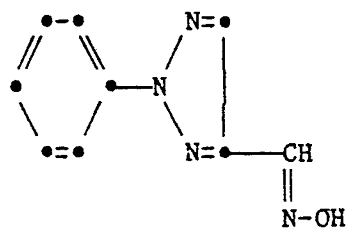

10. Verwendung gemäss Anspruch 1 einer Verbindung der Formel

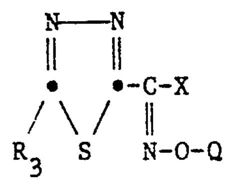

worin $R_3$ $C_1$-$C_4$-Alkoxy, $C_1$-$C_4$-Alkylthio, $C_1$-$C_4$-Alkylsulfinyl, $C_1$-$C_4$-Alkylsulfonyl oder Phenyl, X Wasserstoff, Cyan, Halogen, $C_1$-$C_4$-Alkyl oder $C_1$-$C_4$-Alkoxycarbonyl und Q Wasserstoff oder $C_1$-$C_4$-Alkyl, welches gegebenenfalls durch Cyan substituiert ist, bedeuten.

11. Verwendung gemäss Anspruch 10, worin bei der zu verwendenden Verbindung X Cyan, Halogen oder $C_1$-$C_4$-Alkoxycarbonyl bedeutet.

12. Verwendung gemäss Anspruch 10 einer Verbindung der Formel

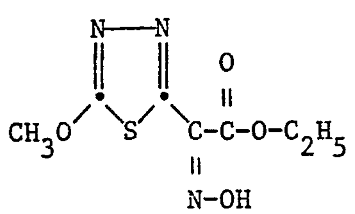

13. Verwendung gemäss Anspruch 1 einer Verbindung der Formel

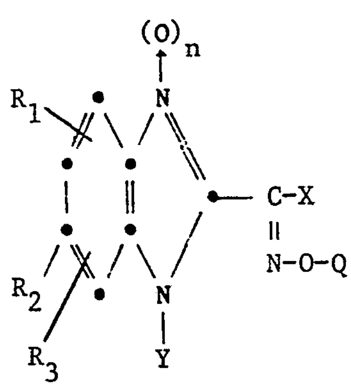

worin n die Zahl 0 oder 1, $R_1$ und $R_3$ Wasserstoff, $R_2$ Wasserstoff oder Halogen, Y $C_1$-$C_4$-Alkyl, $C_1$-$C_4$-

Alkoxycarbonyl oder Phenyl, X Wasserstoff, Cyan, Halogen oder $C_1$-$C_4$-Alkyl und Q Wasserstoff, $C_1$-$C_4$-Alkylcarbonyl, Benzoyl, welches gegebenenfalls durch Halogen substituiert ist, oder Aminocarbonyl, welches am Stickstoffatom gegebenenfalls ein- oder zweimal durch $C_1$-$C_4$-Alkyl, oder einmal durch Phenyl, welches gegebenenfalls Halogen als Substituenten trägt, substituiert ist, bedeuten.

14. Verwendung gemäss Anspruch 13 einer Verbindung der Formel

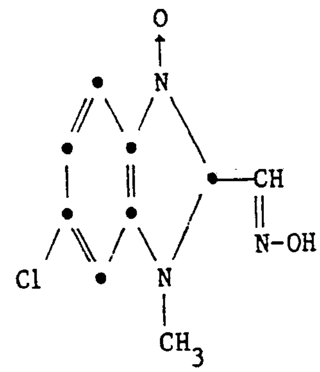

15. Verwendung gemäss Anspruch 13 einer Verbindung der Formel

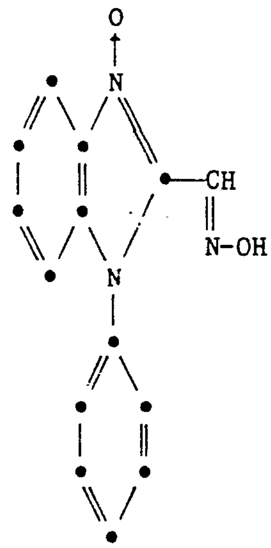

16. Verwendung gemäss Anspruch 1 einer Verbindung der Formel

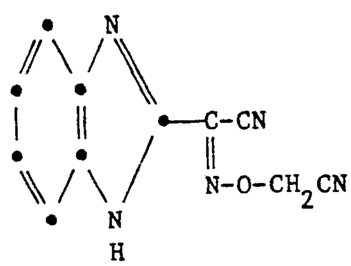

17. Verwendung gemäss Anspruch 13 einer Verbindung der Formel

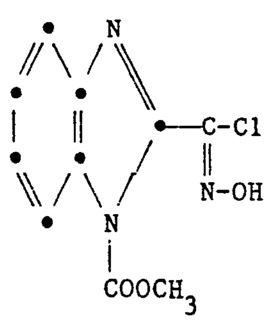

18. Mittel zum Schützen von Kulturpflanzen vor aggressiven Agrarchemikalien enthaltend als Antidote ein Oximderivat der Formel I wie im Anspruch 1 definiert, zusammen mit einem geeigneten Trägermaterial.

19. Antagonistisch wirkende Mittel zum Schützen von Kulturpflanzen vor aggressiven Agrarchemikalien enthaltend als Wirkstoffe a) die Agrarchemikalien sowie b) ein für Kulturpflanzen dazu als Antidote wirkendes Oximderivat der Formel I wie im Anspruch 1 definiert im Mischungsverhältnis 100 : 1 bis 1 : 5, vorzugsweise 20 : 1 bis 1 : 1.

20. Mittel gemäss Anspruch 19, worin die Agrarchemikalie ein Herbizid ist.

21. Mittel gemäss einem der Ansprüche 18 bis 20, welches als Antidot ein Oximderivat, wie in einem der Ansprüche 2 bis 17 definiert, enthält.

22. Mittel gemäss einem der Ansprüche 18 bis 20, welches als Antidot ein Oximderivat aus der nachfolgenden Gruppe

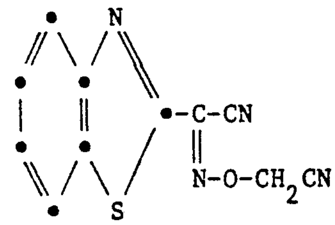

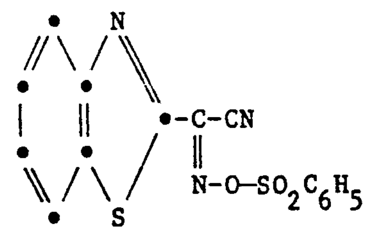

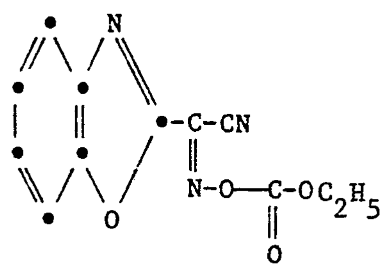

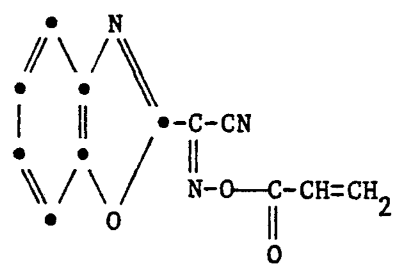

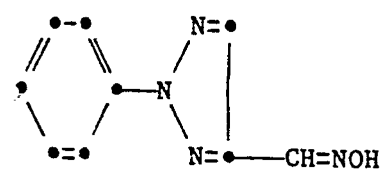

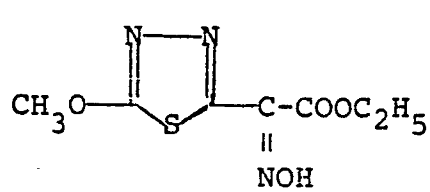

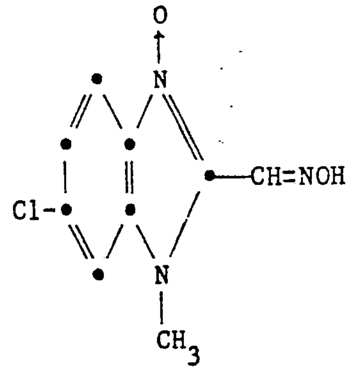

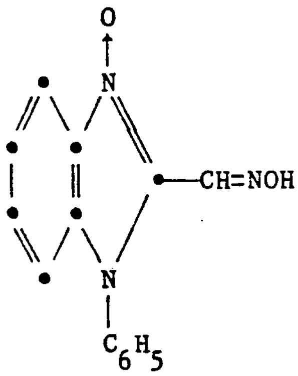

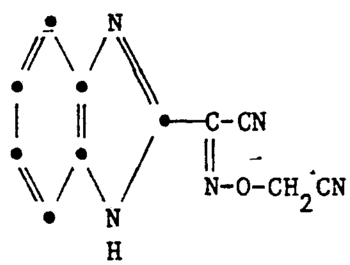

und

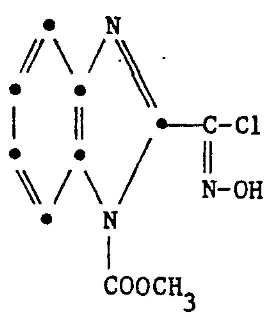

enthält.

23. Vermehrungsgut von Kulturpflanzen, behandelt mit einem Oximderivat der Formel I wie im Anspruch 1 definiert.

24. Vermehrungsgut gemäss Anspruch 23, dadurch gekennzeichnet, dass es sich um Saatgut handelt.

**Claims** (for the Contracting States : BE, CH, DE, FR, GB, IT, NL)

1. Use of compounds of the formula I

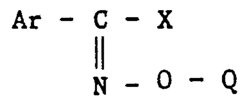 (I)

for protecting cultivated crops against the phytotoxic action of aggressive agricultural chemicals, in which formula

Ar is a five-membered, unsaturated heterocyclic ring which contains 2 or 3 hetero atoms from the group O, S and N, of which at least one is a nitrogen atom, which heterocyclic ring may be fused with a benzene ring, and which can, on the heterocyclic or aromatic part, be substituted by $R_1$, $R_2$ and $R_3$ and/or unsubstituted or substituted by oxo or thioxo, where $R_1$ is hydrogen, halogen, $C_1$-$C_4$-alkyl or $C_1$-$C_4$-alkoxy, $R_2$ is hydrogen, halogen, nitro, $C_1$-$C_4$-alkyl, halogen-$C_1$-$C_4$-alkyl or $C_1$-$C_4$-alkoxy, and $R_3$ is hydrogen or phenyl, or, if the heterocycle is a thiadiazole, $R_1$ and $R_2$ are hydrogen, and $R_3$ is $C_1$-$C_4$-alkoxy, $C_1$-$C_4$-alkylthio, $C_1$-$C_4$-alkylsulfinyl, $C_1$-$C_4$-alkylsulfonyl or phenyl,

X is hydrogen, cyano, halogen, $C_1$-$C_4$-alkyl or $C_1$-$C_4$-alkoxycarbonyl and

Q is hydrogen, a metal cation, $C_1$-$C_4$-alkyl, which can be substituted by cyano or carbamoyl, or is $C_2$-$C_4$-alkenyl, $C_2$-$C_4$-alkynyl, $C_1$-$C_4$-alkyl carbonyl, halogen-$C_1$-$C_4$-alkylcarbonyl, $C_2$-$C_4$-alkenylcarbonyl, $C_1$-$C_4$-alkoxy-carbonyl, $C_1$-$C_4$-alkylthio-carbonyl, $C_1$-$C_4$-alkylsulfonyl, $C_1$-$C_4$-alkoxycarbonyl-$C_1$-$C_4$-alkyl, furanoyl, benzoyl, which is unsubstituted or substituted by one or two substituents of the group methyl, methoxy, halogen or nitro, or is benzylcarbonyl, aminocarbonyl, which is unsubstituted or mono- or disubstituted on the nitrogen atom by $C_1$-$C_4$-alkyl, $C_2$-$C_4$-alkenyl, halogen-$C_1$-$C_4$-alkyl or $C_1$-$C_4$-alkoxy, or is monosubstituted by phenyl which is unsubstituted or carries as substituents trifluoromethyl and/or halogen, or is phenylsulfonyl.

2. Use according to claim 1 of a compound of the formula

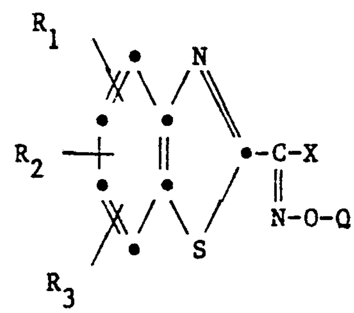

wherein $R_1$, $R_2$ and $R_3$ have the meanings given for formula I, X is cyano or $C_1$-$C_4$-alkoxycarbonyl, and Q is $C_1$-$C_4$-alkyl which can be substituted by cyano, or is $C_2$-$C_4$-alkenyl, $C_2$-$C_4$-alkynyl, $C_1$-$C_4$-alkylcarbonyl, $C_2$-$C_4$-alkenylcarbonyl, $C_1$-$C_4$-alkylsulfonyl, benzoyl, which is unsubstituted or substituted by halogen, or is aminocarbonyl, which is unsubstituted or mono- or disubstituted on the nitrogen atom by $C_1$-$C_4$-alkyl or $C_2$-$C_4$-alkenyl, or is monosubstituted by phenyl which is unsubstituted or carries as substituents trifluoromethyl and/or halogen.

3. Use according to claim 2, wherein in the compound to be used $R_1$, $R_2$ and $R_3$ are hydrogen.

4. Use according to claim 3 of a compound of the formula

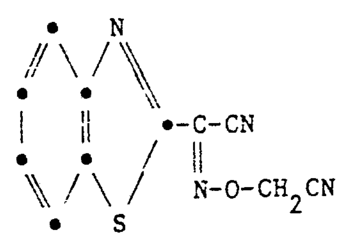

5. Use according to claim 1 of a compound of the formula

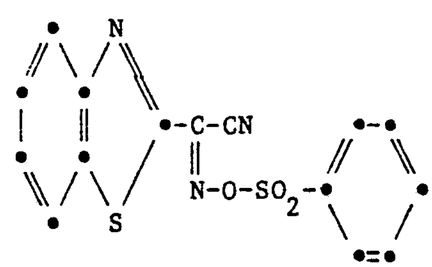

6. Use according to claim 1 of a compound of the formula

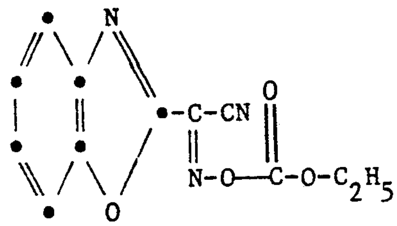

7. Use according to claim 1 of a compound of the formula

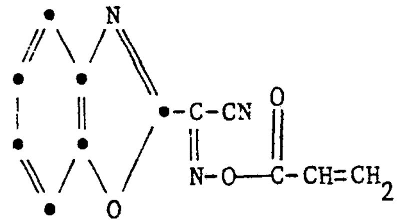

8. Use according to claim 1 of a compound of the formula

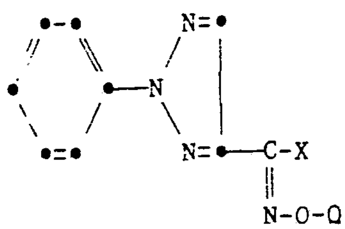

wherein X is hydrogen, and Q is hydrogen or aminocarbonyl which is mono- or disubstituted on the nitrogen atom by $C_1$-$C_4$-alkyl or halogen-$C_1$-$C_4$-alkyl.

9. Use according to claim 8 of a compound of the formula

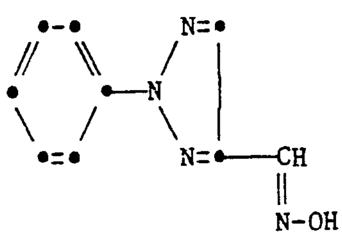

10. Use according to claim 1 of a compound of the formula

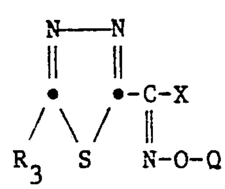

wherein $R_3$ is $C_1$-$C_4$-alkoxy, $C_1$-$C_4$-alkylthio, $C_1$-$C_4$-alkylsulfinyl, $C_1$-$C_4$-alkylsulfonyl or phenyl, X is hydrogen, cyano, halogen, $C_1$-$C_4$-alkylsulfonyl or phenyl, X is hydrogen, cyano, halogen, $C_1$-$C_4$-alkyl or $C_1$-$C_4$-alkoxycarbonyl, and Q is hydrogen or $C_1$-$C_4$-alkyl which is unsubstituted or substituted by cyano.

11. Use according to claim to, wherein in the compound to be used X is cyano, halogen or $C_1$-$C_4$-alkoxycarbonyl.

12. Use according to claim 10 of a compound of the formula

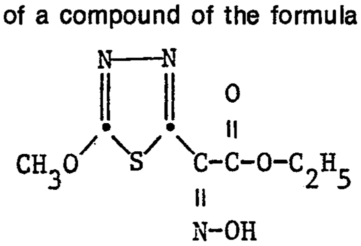

13. Use according to claim 1 of a compound of the formula

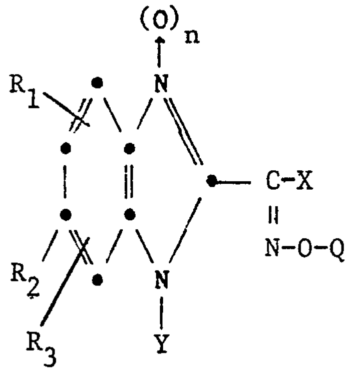

wherein n is the number 0 or 1, $R_1$ and $R_3$ are hydrogen, $R_2$ is hydrogen of halogen, Y is $C_1$-$C_4$-alkyl, $C_1$-$C_4$-alkoxycarbonyl or phenyl, X is hydrogen, cyano, halogen or $C_1$-$C_4$-alkyl, and Q is hydrogen, $C_1$-$C_4$-alkylcarbonyl, benzoyl which is unsubstituted or substituted by halogen, or is aminocarbonyl which is unsubstituted or mono- or disubstituted on the nitrogen atom by $C_1$-$C_4$-alkyl, or is monosubstituted by phenyl which is unsubstituted or carries halogen as substituent.

14. Use according to claim 13 of a compound of the formula

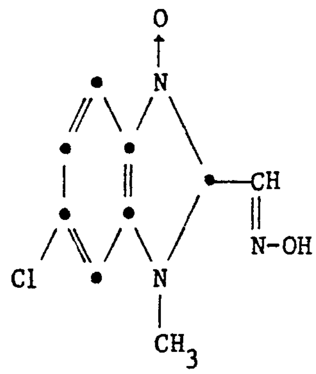

15. Use according to claim 13 of a compound of the formula

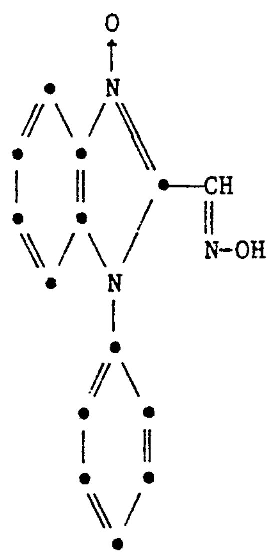

16. Use according to claim 1 of a compound of the formula

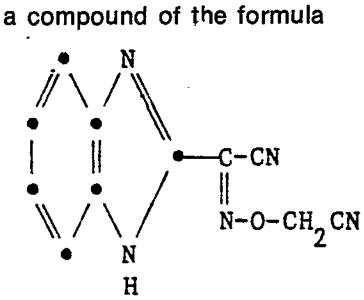

17. Use according to claim 13 of a compound of the formula

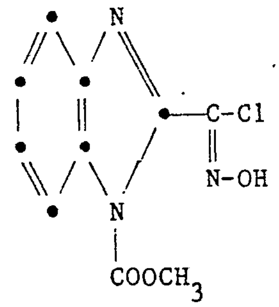

18. A composition for protecting cultivated plants against aggressive agricultural chemicals, which contains, as antidote, an oxime derivative of the formula I as defined in claim 1, together with a suitable carrier material.

19. A composition having an antagonistic action, for protecting cultivated plants against aggressive agricultural chemicals, which contains as active ingredients : a) the agricultural chemicals and b) an oxime derivative of the formula I as defined in claim 1, which acts as an antidote to the agricultural chemicals for cultivated plants, in the mixture ratio of 100 : 1 to 1 : 5, preferably 20 : 1 to 1 : 1.

20. A composition according to claim 19, wherein the agricultural chemical is a herbicide.

21. A generative part of cultivated plants, which part is treated with an oxime derivative of the formula I as defined in claim 1.

22. A generative part according to claim 21, characterised in that the generative part is seed.

23. Oxime derivatives of the formula I as defined in claim 1.

24. An oxime derivative as defined in any one of claims 2 to 17.

25. An oxime derivative as defined in any one of claims 2 to 17.

25. An oxime derivative according to claim 23 from the following group :

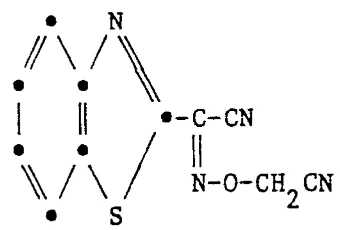

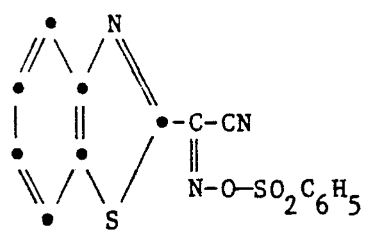

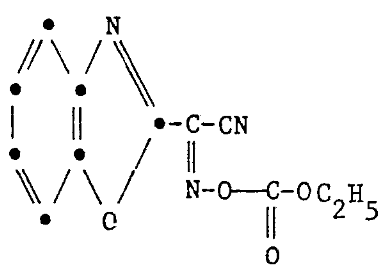

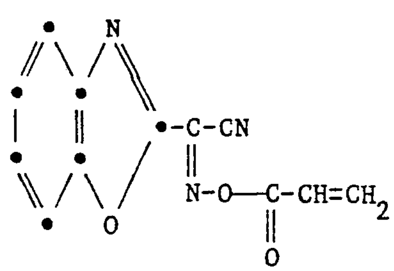

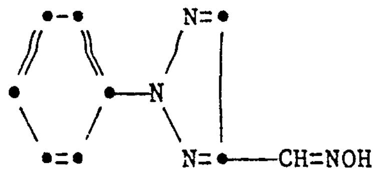

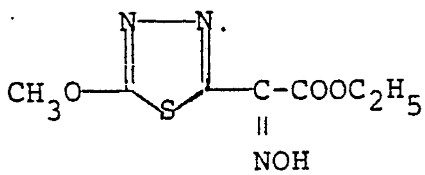

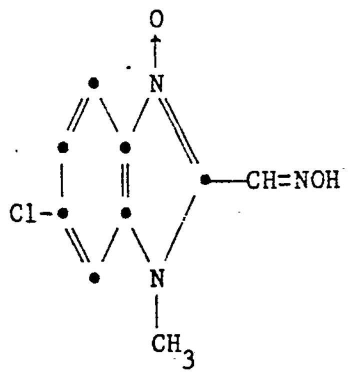

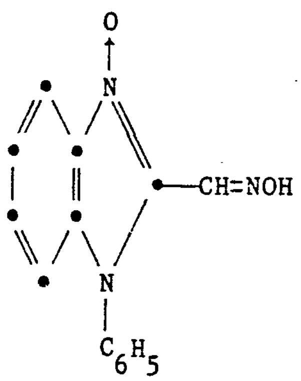

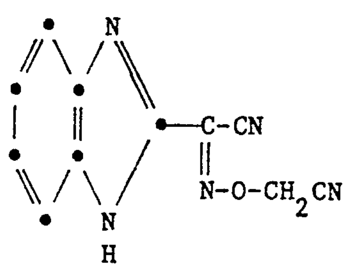

and

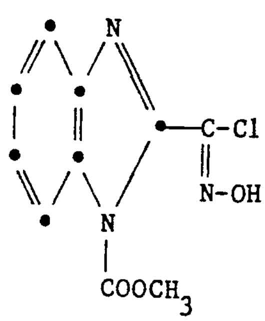

**Claims** (for the Contracting State AT)

1. Use of compounds of the formula I

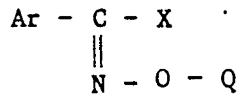 (I)

for protecting cultivated crops against the phytotoxic action of aggressive agricultural chemicals, in which formula

Ar is a five-membered, unsaturated heterocyclic ring which contains 2 or 3 hetero atoms from the group O, S and N, of which at least one is a nitrogen atom, which heterocyclic ring may be fused with a benzene ring, and which can, on the heterocyclic or aromatic part, be substituted by $R_1$, $R_2$ and $R_3$ and/or unsubstituted or substituted by oxo or thioxo, where $R_1$ is hydrogen, halogen, $C_1$-$C_4$-alkyl or $C_1$-$C_4$-alkoxy, $R_2$ is hydrogen, halogen, nitro, $C_1$-$C_4$-alkyl, halogen-$C_1$-$C_4$-alkyl or $C_1$-$C_4$-alkoxy, and $R_3$ is hydrogen or phenyl, or, if the heterocycle is a thiadiazole, $R_1$ and $R_2$ are hydrogen, and $R_3$ is $C_1$-$C_4$-alkoxy, $C_1$-$C_4$-alkylthio, $C_1$-$C_4$-alkylsulfinyl, $C_1$-$C_4$-alkylsulfonyl or phenyl,

X is hydrogen, cyano, halogen, $C_1$-$C_4$-alkyl or $C_1$-$C_4$-alkoxycarbonyl and

Q is hydrogen, a metal cation, $C_1$-$C_4$-alkyl, which can be substituted by cyano or carbamoyl, or is $C_2$-$C_4$-alkenyl, $C_2$-$C_4$-alkynyl, $C_1$-$C_4$-alkyl carbonyl, halogen-$C_1$-$C_4$-alkylcarbonyl, $C_2$-$C_4$-alkenylcarbonyl, $C_1$-$C_4$-alkoxycarbonyl, $C_1$-$C_4$-alkylthio-carbonyl, $C_1$-$C_4$-alkylsulfonyl, $C_1$-$C_4$-alkoxycarbonyl-$C_1$-$C_4$-alkyl, furanoyl, benzoyl, which is unsubstituted or substituted by one or two substituents of the group methyl, methoxy, halogen or nitro, or is benzylcarbonyl, aminocarbonyl, which is unsubstituted or mono- or disubstituted on the nitrogen atom by $C_1$-$C_4$-alkyl, $C_2$-$C_4$-alkenyl, halogen-$C_1$-$C_4$-alkyl or $C_1$-$C_4$-alkoxy, or is monosubstituted by phenyl which is unsubstituted or carries as substituents trifluoromethyl and/or halogen, or is phenylsulfonyl.

2. Use according to claim 1 of a compound of the formula

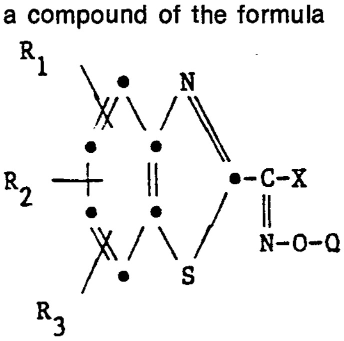

wherein $R_1$, $R_2$ and $R_3$ have the meanings given for formula I, X is cyano or $C_1$-$C_4$-alkoxycarbonyl, and Q is $C_1$-$C_4$-alkyl which can be substituted by cyano, or is $C_2$-$C_4$-alkenyl, $C_2$-$C_4$-alkynyl, $C_1$-$C_4$-alkylcarbonyl, $C_2$-$C_4$-alkenylcarbonyl, $C_1$-$C_4$-alkylsulfonyl, benzoyl, which is unsubstituted or substituted by halogen, or is aminocarbonyl, which is unsubstituted or mono- or disubstituted on the nitrogen atom by $C_1$-$C_4$-alkyl or $C_2$-$C_4$-alkenyl, or is monosubstituted by phenyl which is unsubstituted or carries as substituents trifluoromethyl and/or halogen.

3. Use according to claim 2, wherein in the compound to be used $R_1$, $R_2$ and $R_3$ are hydrogen.

4. Use according to claim 3 of a compound of the formula

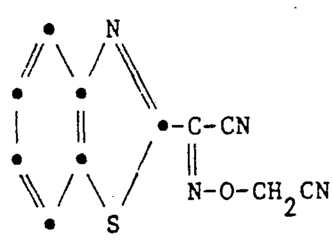

5. Use according to claim 1 of a compound of the formula

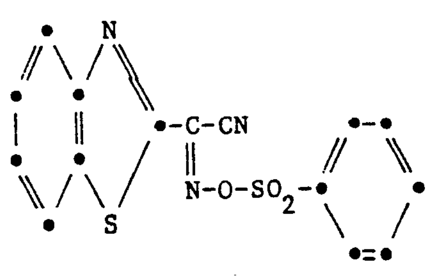

6. Use according to claim 1 of a compound of the formula

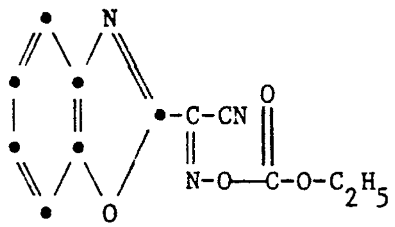

7. Use according to claim 1 of a compound of the formula

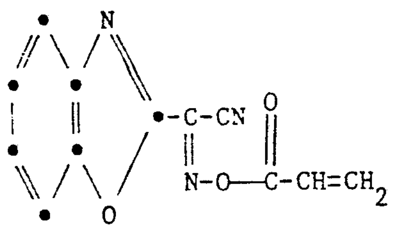

8. Use according to claim 1 of a compound of the formula

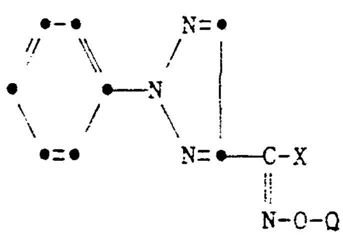

wherein X is hydrogen, and Q is hydrogen or aminocarbonyl which is mono- or disubstituted on the nitrogen atom by $C_1$-$C_4$-alkyl or halogen-$C_1$-$C_4$-alkyl.

9. Use according to claim 8 of a compound of the formula

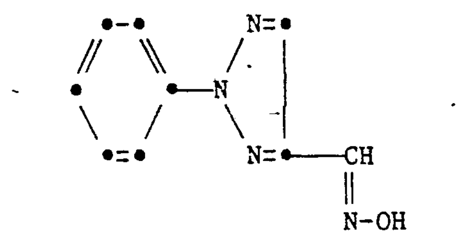

10. Use according to claim 1 of a compound of the formula

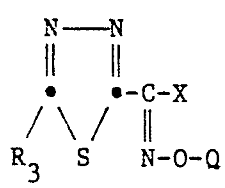

wherein $R_3$ is $C_1$-$C_4$-alkoxy, $C_1$-$C_4$-alkylthio, $C_1$-$C_4$-alkylsulfinyl, $C_1$-$C_4$-alkylsulfonyl or phenyl, X is hydrogen, cyano, halogen, $C_1$-$C_4$-alkyl or $C_1$-$C_4$-alkoxycarbonyl, and Q is hydrogen or $C_1$-$C_4$-alkyl which is unsubstituted or substituted by cyano.

11. Use according to claim 10, wherein in the compound to be used X is cyano, halogen or $C_1$-$C_4$-alkoxycarbonyl.

12. Use according to claim 10 of a compound of the formula

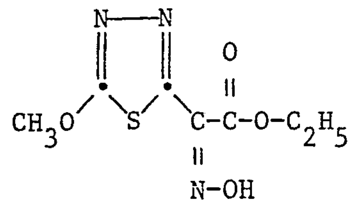

13. Use according to claim 1 of a compound of the formula

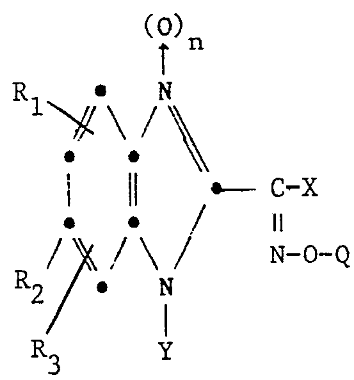

wherein n is the number 0 or 1, $R_1$ and $R_3$ are hydrogen, $R_2$ is hydrogen or halogen, Y is $C_1$-$C_4$-alkyl, $C_1$-$C_4$-alkoxycarbonyl or phenyl, X is hydrogen, cyano, halogen or $C_1$-$C_4$-alkyl, and Q is hydrogen, $C_1$-$C_4$-alkylcarbonyl, benzoyl which is unsubstituted or substituted by halogen, or is aminocarbonyl which is unsubstituted or mono- or disubstituted on the nitrogen atom by $C_1$-$C_4$-alkyl, or is monosubstituted by phenyl which is unsubstituted or carries halogen as substituent.

14. Use according to claim 13 of a compound of the formula

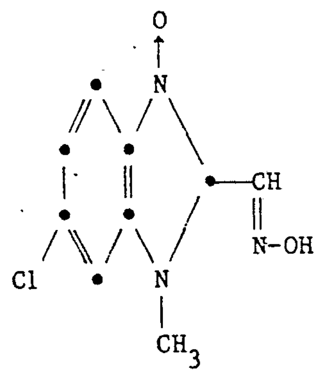

15. Use according to claim 13 of a compound of the formula

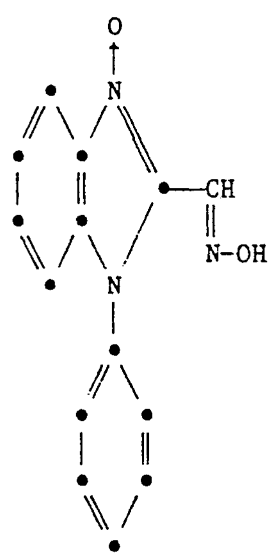

16. Use according to claim 1 of a compound of the formula

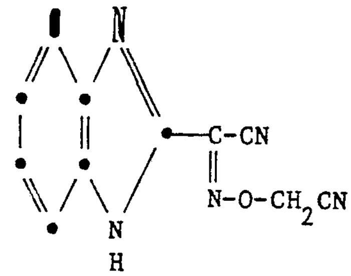

17. Use according to claim 13 of a compound of the formula

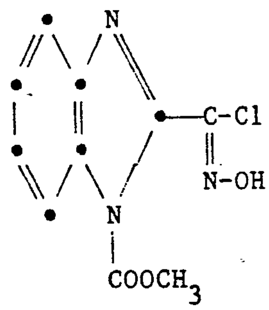

18. A composition for protecting cultivated plants against aggressive agricultural chemicals, which contains, as antidote, an oxime derivative of the formula I as defined in claim 1, together with a suitable carrier material.

19. A composition having an antagonistic action, for protecting cultivated plants against aggressive agricultural chemicals, which contains as active ingredients : a) the agricultural chemicals and b) an oxime derivative of the formula I as defined in claim 1, which acts as an antidote to the agricultural chemicals for cultivated plants, in the mixture ratio of 100 : 1 to 1 : 5, preferably 20 : 1 to 1 : 1.

20. A composition according to claim 19, wherein the agricultural chemical is a herbicide.

21. A composition according to any one of claims 18 to 20, which contains, as antidote, an oxime derivative as defined in any one of claims 2 to 17.

22. A composition according to any one of claims 18 to 20, which contains, as antidote, an oxime derivative from the following group :

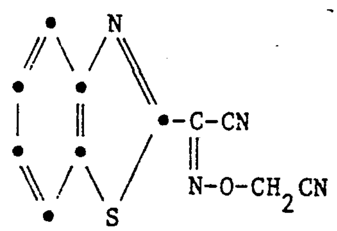

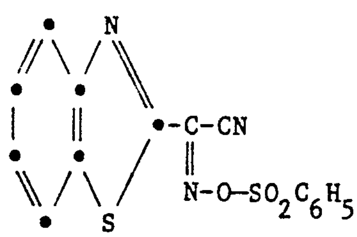

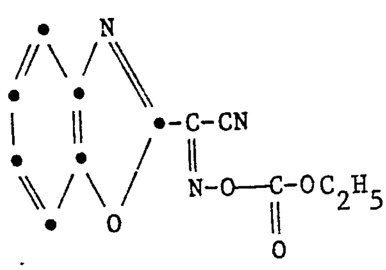

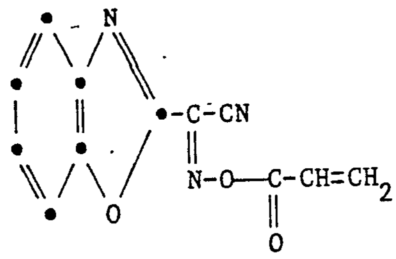

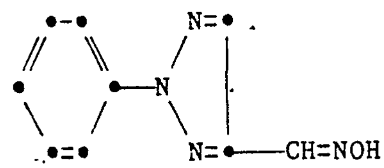

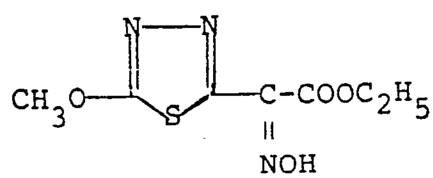

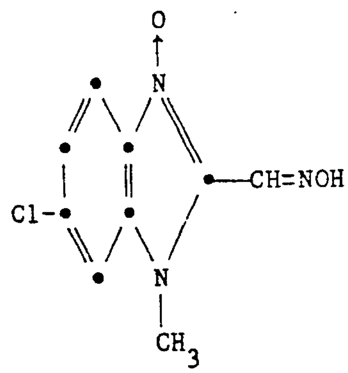

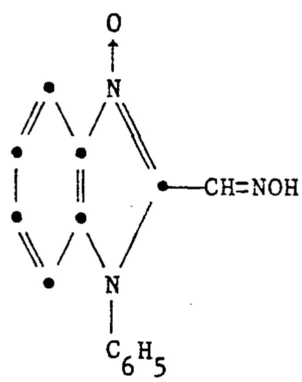

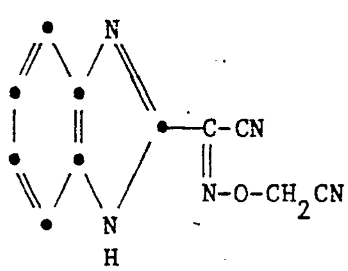

and

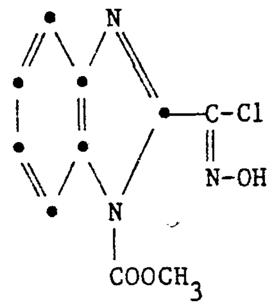

23. A generative part of cultivated plants, which part is treated with an oxime derivative of the formula I as defined in claim 1.

24. A generative part according to claim 23, characterised in that the generative part is seed.

**Revendications** (pour les Etats Contractants : BE, CH, DE, FR, GB, IT, NL)

1. Application de composés de formule I

$$Ar - \underset{\underset{N-O-Q}{\|}}{C} - X \qquad (I)$$

à la protection des cultures de plantes contre l'action phytotoxique des produits agrochimiques agressifs, formule dans laquelle

Ar représente un noyau hétérocyclique à 5 chaînons, non saturé, le cas échéant avec condensation benzénique, qui contient 2 ou 3 hétéroatomes du groupe O, S et N dont au moins un est un atome d'azote, qui est substitué sur la fraction hétérocyclique ou aromatique par $R_1$, $R_2$ et $R_3$ et/ou qui est éventuellement substitué par un oxo ou un thioxo, où $R_1$ représente un hydrogène, un halogène, un alcoyle en $C_1$ à $C_4$ ou un alcoxy en $C_1$ à $C_4$, $R_2$ représente un hydrogène, un halogène, un nitro, un alcoyle en $C_1$ à $C_4$, un halogène-alcoyle en $C_1$ à $C_4$ ou un alcoxy en $C_1$ à $C_4$ et $R_3$ représente un hydrogène ou un phényle ou, lorsqu'on a comme hétérocycle un thiadiazole, $R_1$ et $R_2$ représentent un hydrogène et $R_3$ représente un alcoxy en $C_1$ à $C_4$, un alcoylthio en $C_1$ à $C_4$, un alcoyle en $C_1$ à $C_4$-sulfinyle, un alcoyle en $C_1$ à $C_4$-sulfonyle ou un phényle.

X représente un hydrogène, un cyano, un halogène, un alcoyle en $C_1$ à $C_4$ ou un alcoxy en $C_1$ à $C_4$-carbonyle, et

Q représente un hydrogène, un cation métallique, un alcoyle en $C_1$ à $C_4$, qui est éventuellement substitué par un cyano ou un carbamoyle, un alcényle en $C_2$ à $C_4$, un alcynyle en $C_2$ à $C_4$, un alcoyle en $C_1$ à $C_4$-carbonyle, un halogène-alcoyle en $C_1$ à $C_4$-carbonyle, un alcényle en $C_2$ à $C_4$-carbonyle, un alcoxy en $C_1$ à $C_4$-carbonyle, un alcoylthio en $C_1$ à $C_4$-carbonyle, un alcoyle en $C_1$ à $C_4$-sulfonyle, un alcoxy en $C_1$ à $C_4$-carbonyl-alcoyle en $C_1$ à $C_4$, un furanoyle, un benzoyle, qui est éventuellement mono à disubstitué par des substituants du groupe méthyle, méthoxy, halogène ou nitro, un benzylcarbonyle, un aminocarbonyle qui est éventuellement mono- ou disubstitué sur l'atome d'azote par un alcoyle en $C_1$ à $C_4$, un alcényle en $C_2$ à $C_4$, un halogène-alcoyle en $C_1$ à $C_4$ ou un alcoxy en $C_1$ à $C_4$, ou monosubstitué par un phényle, qui porte le cas échéant comme substituants un trifluorométhyle et/ou un halogène, ou représente un phénylsulfonyle.

2. Application selon la revendication 1 d'un composé de formule

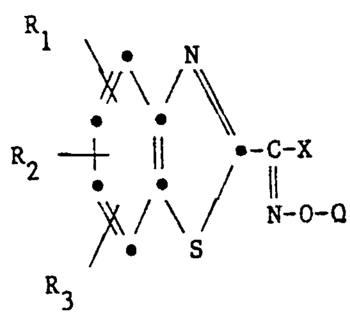

où $R_1$, $R_2$ et $R_3$ ont les significations données pour la formule I, X représente un cyano ou un alcoxy en $C_1$ à $C_4$-carbonyle et Q représente un alcoyle en $C_1$ à $C_4$ qui est éventuellement substitué par un cyano, ou représente un alcényle en $C_2$ à $C_4$, un alcynyle en $C_2$ à $C_4$, un alcoyle en $C_1$ à $C_4$-carbonyle, un alcényle

en $C_2$ à $C_4$-carbonyle, un alcoyle en $C_1$ à $C_4$-sulfonyle, un benzoyle, qui est éventuellement substitué par un halogène, ou représente un aminocarbonyle qui est éventuellement mono- ou disubstitué sur l'atome d'azote par un alcoyle en $C_1$ à $C_4$ ou un alcényle en $C_2$ à $C_4$ ou qui est monosubstitué par un phényle, lequel porte le cas échéant comme substituants un trifluorométhyle et/ou un halogène.

3. Application selon la revendication 2 où dans le composé à utiliser $R_1$, $R_2$ et $R_3$ représentent un hydrogène.

4. Application selon la revendication 3 d'un composé de formule

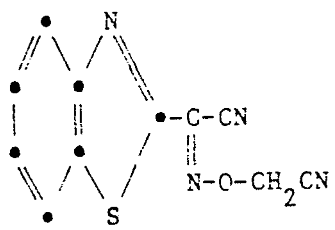

5. Application selon la revendication 1 d'un composé de formule

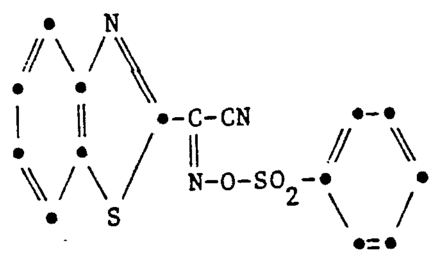

6. Application selon la revendication 1 d'un composé de formule

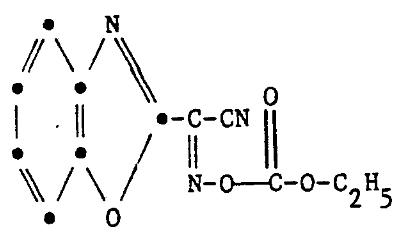

7. Application selon la revendication 1 d'un composé de formule

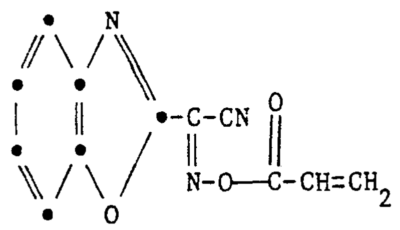

8. Application selon la revendication 1 d'un composé de formule

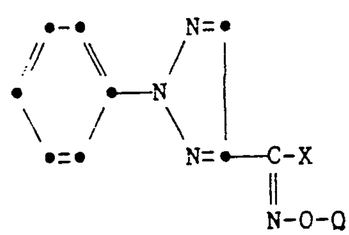

où X représente un hydrogène et Q représente un hydrogène ou un aminocarbonyle qui est mono- ou disubstitué sur l'atome d'azote par un alcoyle en $C_1$ à $C_4$ ou un halogène-alcoyle en $C_1$ à $C_4$.

9. Application selon la revendication 8 d'un composé de formule

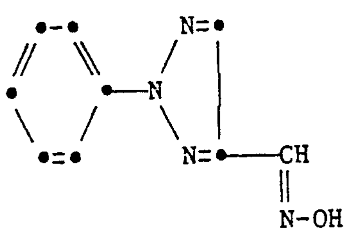

10. Application selon la revendication 1 d'un composé de formule

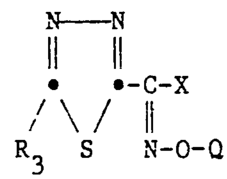

où $R_3$ représente un alcoxy en $C_1$ à $C_4$, un alcoylthio en $C_1$ à $C_4$, un alcoyle en $C_1$ à $C_4$-sulfinyle, un alcoyle en $C_1$ à $C_4$-sulfonyle ou un phényle, X représente un hydrogène, un cyano, un halogène, un alcoyle en $C_1$ à $C_4$ ou un alcoxy en $C_1$ à $C_4$-carbonyle et Q représente un hydrogène ou un alcoyle en $C_1$ à $C_4$ qui est éventuellement substitué par un cyano.

11. Application selon la revendication 10 où dans le composé à utiliser X représente un cyano, un halogène ou un alcoxy en $C_1$ à $C_4$-carbonyle.

12. Application selon la revendication 10 d'un composé de formule

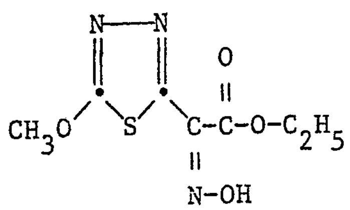

13. Application selon la revendication 1 d'un composé de formule

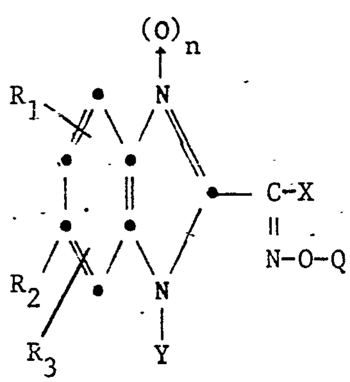

où n représente le nombre 0 ou 1, $R_1$ et $R_3$ représentent un hydrogène, $R_2$ représente un hydrogène ou un halogène, Y représente un alcoyle en $C_1$ à $C_4$, un alcoxy en $C_1$ à $C_4$-carbonyle ou un phényle, X représente un hydrogène, un cyano, un halogène ou un alcoyle en $C_1$ à $C_4$ et Q représente un hydrogène, un alcoyle en $C_1$ à $C_4$-carbonyle, un benzoyle qui est éventuellement substitué par un halogène, ou un aminocarbonyle qui est éventuellement mono-ou disubstitué par un alcoyle en $C_1$ à $C_4$ ou monosubstitué par un phényle qui porte le cas échéant comme substituants des halogènes.

14. Application selon la revendication 13 d'un composé de formule

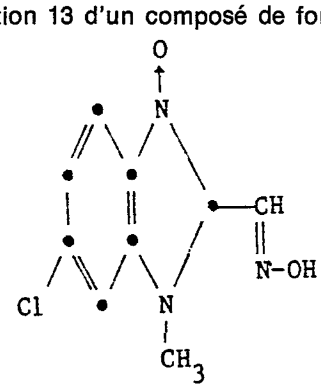

15. Application selon la revendication 13 d'un composé de formule

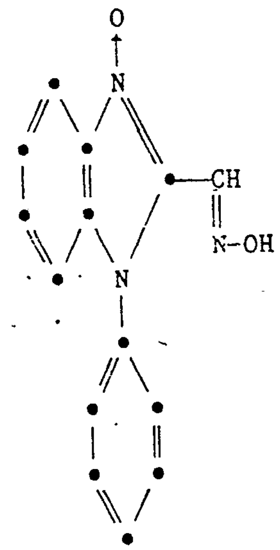

16. Application selon la revendication 1 d'un composé de formule

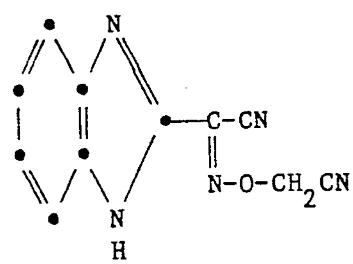

17. Application selon la revendication 13 d'un composé de formule

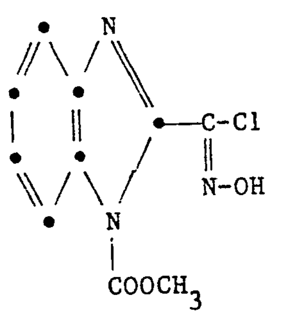

18. Agent de protection des cultures de plantes contre les produits agrochimiques agressifs contenant comme antidote un dérivé d'oxime de formule I telle que définie dans la revendication 1, avec un support approprié.

19. Agent à action antagoniste pour protéger les cultures de plantes contre les produits agrochimiques agressifs contenant comme matières actives a) les produits agrochimiques, ainsi que b) un dérivé d'oxime de formule I tel que défini dans la revendication 1 agissant comme antidote de ce produit pour les cultures de plantes, dans un rapport de mélange de 100 : 1 à 1 : 5, de préférence à 20 : 1 à 1 : 1.

20. Agent selon la revendication 19, où le produit agrochimique est un herbicide.

21. Facteur de multiplication des cultures de plantes, traité avec un dérivé d'oxime de formule I telle que définie dans la revendication 1.

22. Facteur de multiplication selon la revendication 22, caractérisé en ce qu'il s'agit de semences.

23. Dérivés d'oxime de formule I telle que définie dans la revendication 1.

24. Dérivés d'oxime tels que définis dans l'une des revendications 2 à 17.

25. Dérivé d'oxime selon la revendication 23 appartenant au groupe suivant :

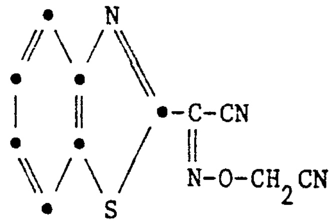

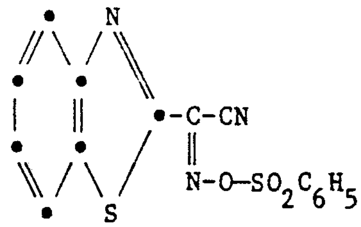

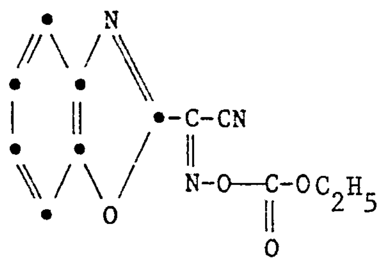

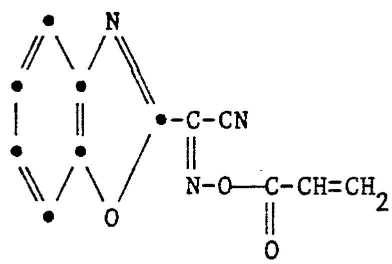

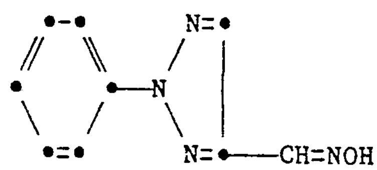

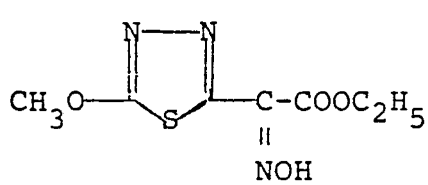

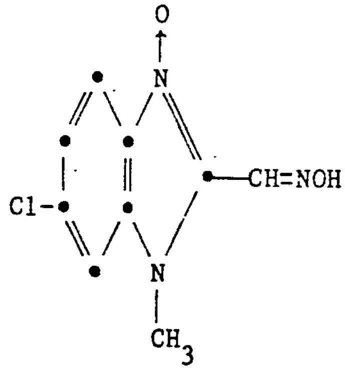

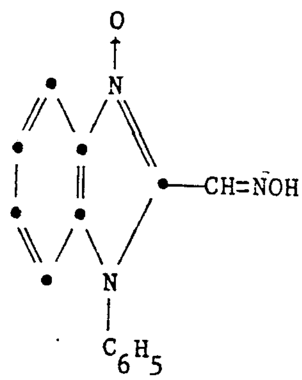

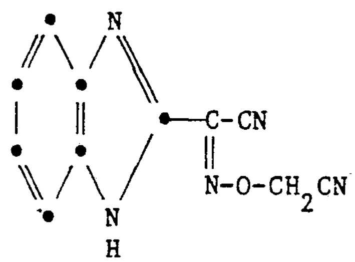

et

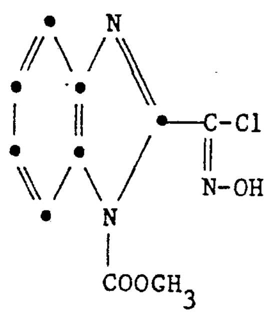

**Revendications** (pour l'Etat contractant AT)

1. Application de composés de formule I

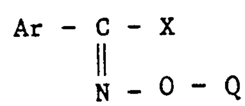

(I)

à la protection des cultures de plantes contre l'action phytotoxique des produits agrochimiques agressifs, formule dans laquelle

Ar représente un noyau hétérocyclique à 5 chaînons, non saturé, le cas échéant avec condensation benzénique, qui contient 2 ou 3 hétéroatomes du groupe O, S et N dont au moins un est un atome d'azote, qui est substitué sur la fraction hétérocyclique ou aromatique par $R_1$, $R_2$ et $R_3$ et/ou qui est éventuellement substitué par un oxo ou un thioxo, où $R_1$ représente un hydrogène, un halogène, un alcoyle en $C_1$ à $C_4$ ou un alcoxy en $C_1$ à $C_4$, $R_2$ représente un hydrogène, un halogène, un nitro, un alcoyle en $C_1$ à $C_4$, un halogène-alcoyle en $C_1$ à $C_4$ ou un alcoxy en $C_1$ à $C_4$ et $R_3$ représente un hydrogène ou un phényle ou, lorsqu'on a comme hétérocycle un thiadiazole, $R_1$ et $R_2$ représentent un hydrogène et $R_3$ représente un alcoxy en $C_1$ à $C_4$, un alcoylthio en $C_1$ à $C_4$, un alcoyle en $C_1$ à $C_4$-sulfinyle, un alcoyle en $C_1$ à $C_4$-sulfonyle ou un phényle,

X représente un hydrogène, un cyano, un halogène, un alcoyle en $C_1$ à $C_4$ ou un alcoxy en $C_1$ à $C_4$-carbonyle, et

Q représente un hydrogène, un cation métallique, un alcoyle en $C_1$ à $C_4$, qui est éventuellement substitué par un cyano ou un carbamoyle, un alcényle en $C_2$ à $C_4$, un alcynyle en $C_2$ à $C_4$, un alcoyle en $C_1$ à $C_4$-carbonyle, un halogène-alcoyle en $C_1$ à $C_4$-carbonyle, un alcényle en $C_2$ à $C_4$-carbonyle, un alcoxy en $C_1$ à $C_4$-carbonyle, un alcoylthio en $C_1$ en $C_1$ à $C_4$-carbonyle, un alcoyle en $C_1$ à $C_4$-sulfonyle, un alcoxy en $C_1$ à $C_4$-carbonyl-alcoyle en $C_1$ à $C_4$, un furanoyle, un benzoyle, qui est éventuellement mono a disubstitué par des substituants du groupe méthyle, méthoxy, halogène ou nitro, un benzylcarbonyle, un aminocarbonyle qui est éventuellement mono- ou disubstitué sur l'atome d'azote par un alcoyle

en $C_1$ à $C_4$, un alcényle en $C_2$ à $C_4$, un halogène-alcoyle en $C_1$ à $C_4$ ou un alcoxy en $C_1$ à $C_4$ ou monosubstitué par un phényle, qui porte le cas échéant comme substituants un trifluorométhyle et/ou un halogène, ou représente un phénylsulfonyle.

2. Application selon la revendication 1 d'un composé de formule

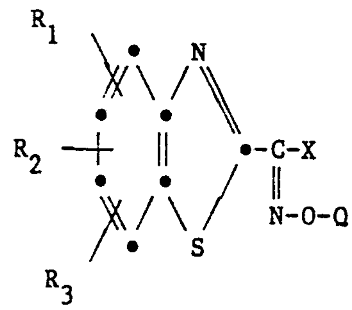

où $R_1$, $R_2$ et $R_3$ ont les significations données pour la formule I, X représente un cyano ou un alcoxy en $C_1$ à $C_4$-carbonyle et Q représente un alcoyle en $C_1$ à $C_4$ qui est éventuellement substitué par un cyano, ou représente un alcényle en $C_2$ à $C_4$, un alcynyle en $C_2$ à $C_4$, un alcoyle en $C_1$ à $C_4$-carbonyle, un alcényle en $C_2$ à $C_4$-carbonyle, un alcoyle en $C_1$ à $C_4$-sulfonyle, un benzoyle, qui est éventuellement substitué par un halogène, ou représente un aminocarbonyle qui est éventuellement mono- ou disubstitué sur l'atome d'azote par un alcoyle en $C_1$ à $C_4$ ou un alcényle en $C_2$ à $C_4$ ou qui est monosubstitué par un phényle, lequel porte le cas échéant comme substituants un trifluorométhyle et/ou un halogène.

3. Application selon la revendication 2 où le composé à utiliser $R_1$, $R_2$ et $R_3$ représentent un hydrogène.

4. Application selon la revendication 3 d'un composé de formule

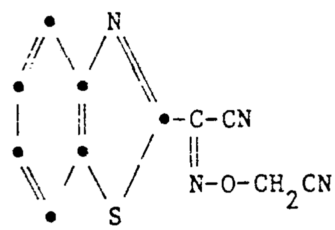

5. Application selon la revendication 1 d'un composé de formule

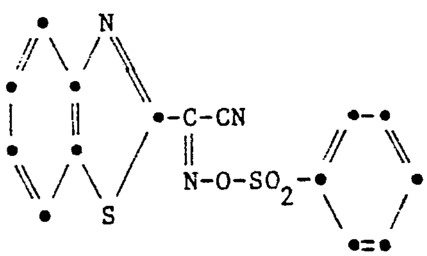

6. Application selon la revendication 1 d'un composé de formule

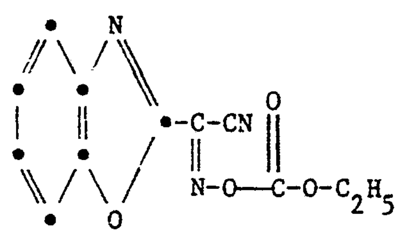

7. Application selon la revendication 1 d'un composé de formule

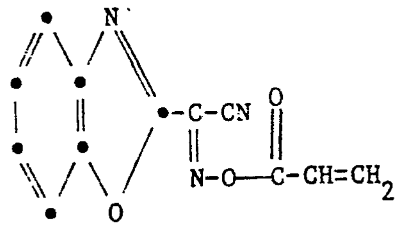

8. Application selon la revendication 1 d'un composé de formule

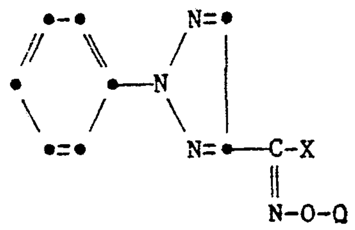

où X représente un hydrogène et Q représente un hydrogène ou un aminocarbonyle qui est mono- ou disubstitué sur l'atome d'azote par un alcoyle en $C_1$ à $C_4$ ou un halogène-alcoyle en $C_1$ à $C_4$.

9. Application selon la revendication 8 d'un composé de formule

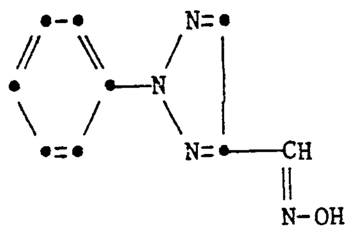

10. Application selon la revendication 1 d'un composé de formule

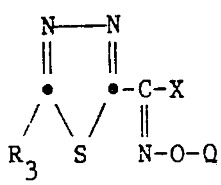

où $R_3$ représente un alcoxy en $C_1$ à $C_4$, un alcoylthio en $C_1$ à $C_4$, un alcoyle en $C_1$ à $C_4$-sulfinyle, un alcoyle en $C_1$ à $C_4$-sulfonyle ou un phényle, X représente un hydrogène, un cyano, un halogène, un alcoyle en $C_1$ à $C_4$ ou un alcoxy en $C_1$ à $C_4$-carbonyle et Q représente un hydrogène ou un alcoyle en $C_1$ à $C_4$ qui est éventuellement substitué par un cyano.

11. Application selon la revendication 10 où dans le composé à utiliser X représente un cyano, un halogène ou un alcoxy en $C_1$ à $C_4$-carbonyle.

12. Application selon la revendication 10 d'un composé de formule

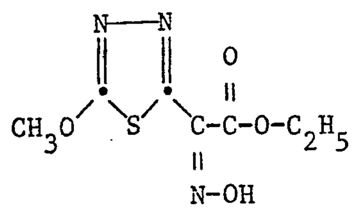

13. Application selon la revendication 1 d'un composé de formule

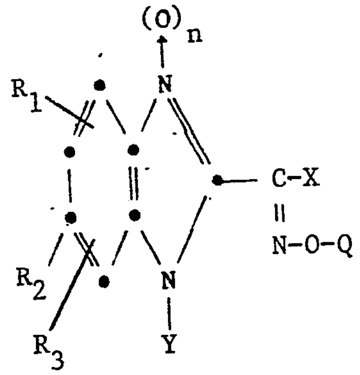

où n représente le nombre 0 ou 1, $R_1$ et $R_3$ représentent un hydrogène, $R_2$ représente un hydrogène ou un halogène, Y représente un alcoyle en $C_1$ à $C_4$, un alcoxy en $C_1$ à $C_4$-carbonyle ou un phényle, X représente un hydrogène, un cyano, un halogène ou un alcoyle en $C_1$ à $C_4$ et Q représente un hydrogène, un alcoyle en $C_1$ à $C_4$-carbonyle, un benzoyle qui est éventuellement substitué par un halogène, ou un aminocarbonyle qui est éventuellement mono- ou disubstitué par un alcoyle en $C_1$ à $C_4$ ou monosubstitué par un phényle qui porte le cas échéant comme substituants des halogènes.

14. Application selon la revendication 13 d'un composé de formule

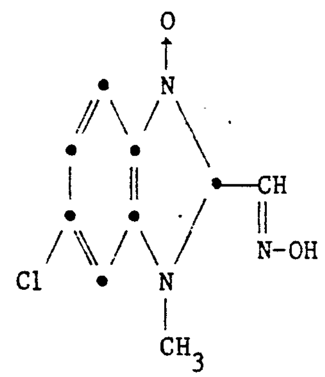

15. Application selon la revendication 13 d'un composé de formule

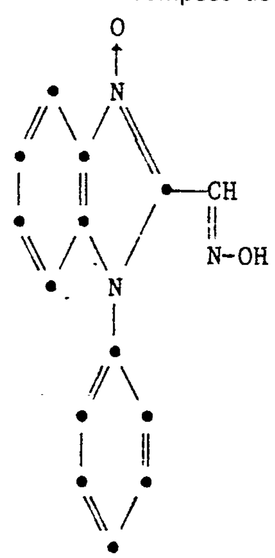

16. Application selon la revendication 1 d'un composé de formule

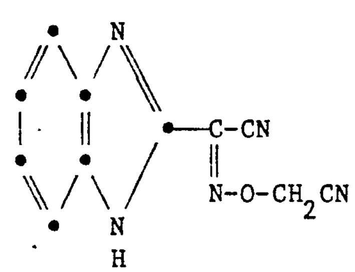

17. Application selon la revendication 13 d'un composé de formule

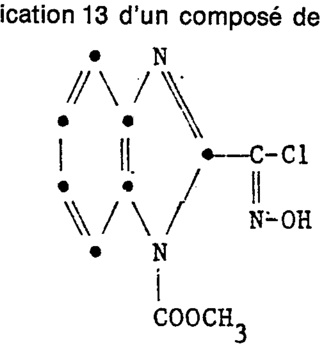

18. Agent de protection des cultures de plantes contre les produits agrochimiques agressifs contenant comme antidote un dérivé d'oxime de formule I telle que définie dans la revendication 1, avec un support approprié.

19. Agent à action antagoniste pour protéger les cultures de plantes contre les produits agrochimiques agressifs contenant comme matières actives a) les produits agrochimiques, ainsi que b) un dérivé d'oxime de formule I tel que défini dans la revendication 1 agissant comme antidote de ce produit pour les cultures de plantes, dans un rapport de mélange de 100 : 1 à 1 : 5, de préférence à 20 : 1 à 1 : 1.

20. Agent selon la revendication 19 où le produit agrochimique est un herbicide.

21. Agent selon l'une des revendications 18 à 20, qui contient comme antidote un dérivé d'oxime tel que défini dans l'une des revendications 2 à 17.

22. Agent selon l'une des revendications 18 à 20 qui contient comme antidote un dérivé d'oxime appartenant au groupe suivant :

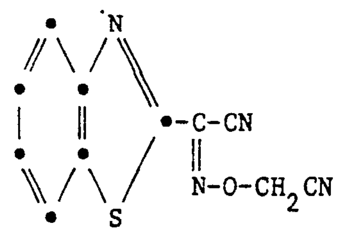

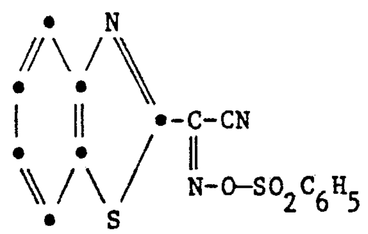

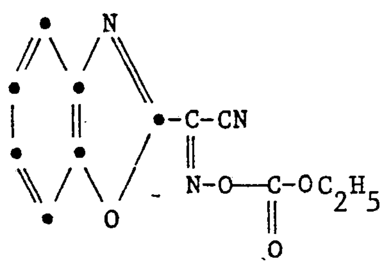

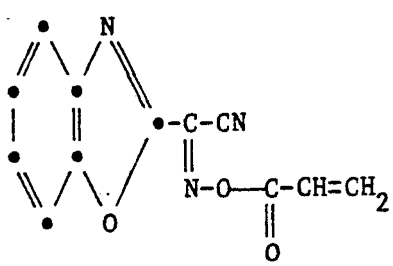

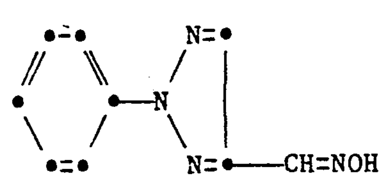

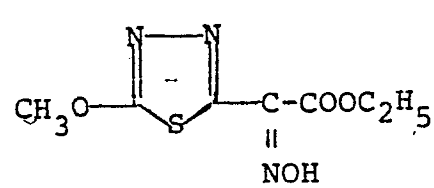

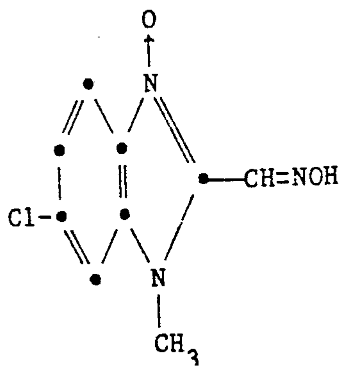

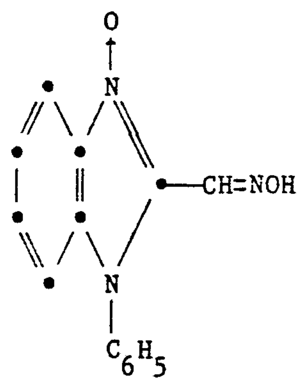

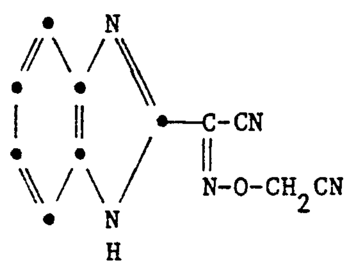

et

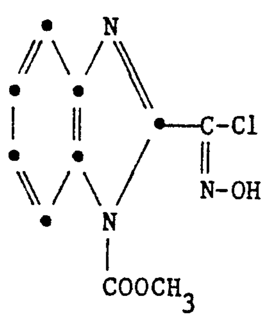

23. Facteur de multiplication des cultures de plantes traité avec un dérivé d'oxime de formule I selon la revendication 1.

24. Facteur de multiplication selon la revendication 23, caractérisé en ce qu'il s'agit de semences.